# EUROPEAN PATENT SPECIFICATION

(11) **EP 2 318 033 B1**
(45) Date of publication and mention of the grant of the patent: **17.10.2018**
(21) Application number: 09777419.4
(22) Date of filing: 24.07.2009
(51) Int. Cl.: A61K 38/17, A61P 29/00

(54) **COMPOSITIONS FOR THE TREATMENT OF PAIN AND/OR INFLAMATION**
ZUSAMMENSETZUNGEN ZUR BEHANDLUNG VON SCHMERZEN UND/ODER ENTZÜNDUNGEN
COMPOSITIONS DESTINÉES AU TRAITEMENT DE LA DOULEUR ET/OU DE L'INFLAMMATION

(30) Priority: 24.07.2008 ES 200802210
(43) Date of publication of application: 11.05.2011
(73) Proprietor: BCN Peptides, S.A., 08777 Sant Quintí de Mediona (ES)
(72) Inventor: CARRENO SERRAÏMA, Cristina, 08015 Barcelona (ES); VAN DEN NEST, Wim, 08800 Vilanova I La Geltrú - Barcelona (ES); FERRER MONTIEL, Antonio, 03540 Playa de San Juan-Alicante (ES); CAMPRUBÍ ROBLES, Maria, 03540 Playa de San Juan-Alicante (ES); FERNÁNDEZ CARNEADO, Jimena, 33300 Villaviciosa (Asturias) (ES); PONSATI OBIOLS, Berta, 08005 Barcelona (ES)
(74) Representative: Carvajal y Urquijo, Isabel
(86) International application number: PCT/EP2009/005381
(87) International publication number: WO 2010/009892

(56) References cited:
- WO-A1-97/34620
- WO-A1-02/089834
- WO-A2-01/78760
- WO-A2-2007/093807
- DATABASE WPI Week 200951 Thomson Scientific, London, GB; AN 2009-J28980 XP002573254 & KR 2009 041 066 A (UNIV SUNGKYUNKWAN FOUND CORP COLLABORATI) 28 April 2009 (2009-04-28)

### SEQUENCE LISTING

<110> BCN PEPTIDES, S.A.
<120> COMPOSITIONS FOR THE TREATMENT OF PAIN AND/OR INFLAMMATION
<130> DD-BCN2-
<160> 32
<170> PatentIn version 3.3
<210> 1
   <211> 20̸6
   <212> PRT
   <213> Homo sapiens
<400> 1
<210> 2
   <211> 84
   <212> PRT
   <213> Homo sapiens
<400> 2
   Met Ala Glu Asp Ala Asp Met Arg Asn Glu Leu Glu Glu Met Gln Arg
   1 5 10 15
   Arg Ala Asp Gln Leu Ala Asp Glu Ser Leu Glu Ser Thr Arg Arg Met
   20 25 30
   Leu Gln Leu Val Glu Glu Ser Lys Asp Ala Gly Ile Arg Thr Leu Val
   35 40 45
   Met Leu Asp Glu Gln Gly Glu Gln Leu Glu Arg Ile Glu Glu Gly Met
   50 55 60
   Asp Gln Ile Asn Lys Asp Met Lys Glu Ala Glu Lys Asn Leu Thr Asp
   65 70 75 80
   Leu Gly Lys Phe
<210> 3
   <211> 37
   <212> PRT
   <213> Homo sapiens
<400> 3
   Glu Ile Asp Thr Gln Asn Arg Gln Ile Asp Arg Ile Met Glu Lys Ala
   1 5 10 15
   Asp Ser Asn Lys Thr Arg Ile Asp Glu Ala Asn Gln Arg Ala Thr Lys
   20 25 30
   Met Leu Gly Ser Gly
   35
<210> 4
   <211> 13
   <212> PRT
   <213> Homo sapiens
<400> 4
   Glu Leu Glu Glu Met Gln Arg Arg Ala Asp Gln Leu Ala
   1 5 10
<210> 5
   <211> 16
   <212> PRT
   <213> Homo sapiens
<400> 5
   Ser Leu Glu Ser Thr Arg Arg Met Leu Gln Leu Val Glu Glu Ser Lys
   1               5                   10                  15
<210> 6
   <211> 17
   <212> PRT
   <213> Homo sapiens
<400> 6
   Asp Gln Ile Asn Lys Asp Met Lys Glu Ala Glu Lys Asn Leu Thr Asp
   1               5                   10                  15
   Leu
<210> 7
   <211> 26
   <212> PRT
   <213> Homo sapiens
<400> 7
   Ile Met Glu Lys Ala Asp Ser Asn Lys Thr Arg Ile Asp Glu Ala Asn
   1               5                   10                  15
   Gln Arg Ala Thr Lys Met Leu Gly Ser Gly
               20                  25
<210> 8
   <211> 8
   <212> PRT
   <213> Homo sapiens
<400> 8
   Glu Glu Met Gln Arg Arg Ala Asp
   1               5
<210> 9
   <211> 13
   <212> PRT
   <213> Homo sapiens
<400> 9
   Leu Glu Ser Thr Arg Arg Met Leu Gln Leu Val Glu Glu
   1 5 10
<210> 10
   <211> 12
   <212> PRT
   <213> Homo sapiens
<400> 10
   Asn Lys Asp Met Lys Glu Ala Glu Lys Asn Leu Thr
   1 5 10
<210> 11
   <211> 6
   <212> PRT
   <213> Homo sapiens
<400> 11
   Glu Glu Met Gln Arg Arg
   1 5
<210> 12
   <211> 21
   <212> PRT
   <213> Homo sapiens
<400> 12
   Met Ala Glu Asp Ala Asp Met Arg Asn Glu Leu Glu Glu Met Gln Arg
   1 5 10 15
   Arg Ala Asp Gln Leu
   20
<210> 13
   <211> 23
   <212> PRT
   <213> Homo sapiens
<400> 13
   Ala Asp Glu Ser Leu Glu Ser Thr Arg Arg Met Leu Gln Leu Val Glu
   1 5 10 15
   Glu Ser Lys Asp Ala Gly Ile
   20
<210> 14
   <211> 12
   <212> PRT
   <213> Homo sapiens
<400> 14
   Glu Leu Glu Glu Met Gln Arg Arg Ala Asp Gln Leu
   1               5                   10
<210> 15
   <211> 11
   <212> PRT
   <213> Homo sapiens
<400> 15
   Glu Leu Glu Glu Met Gln Arg Arg Ala Asp Gln
   1               5                   10
<210> 16
   <211> 10
   <212> PRT
   <213> Homo sapiens
<400> 16
   Glu Leu Glu Glu Met Gln Arg Arg Ala Asp
   1               5                   10
<210> 17
   <211> 9
   <212> PRT
   <213> Homo sapiens
<400> 17
   Glu Leu Glu Glu Met Gln Arg Arg Ala
   1               5
<210> 18
   <211> 8
   <212> PRT
   <213> Homo sapiens
<400> 18
   Glu Leu Glu Glu Met Gln Arg Arg
   1               5
<210> 19
   <211> 11
   <212> PRT
   <213> Homo sapiens
<400> 19
   Leu Glu Glu Met Gln Arg Arg Ala Asp Gln Leu
   1 5 10
<210> 20
   <211> 10
   <212> PRT
   <213> Homo sapiens
<400> 20
   Leu Glu Glu Met Gln Arg Arg Ala Asp Gln
   1 5 10
<210> 21
   <211> 9
   <212> PRT
   <213> Homo sapiens
<400> 21
   Leu Glu Glu Met Gln Arg Arg Ala Asp
   1 5
<210> 22
   <211> 8
   <212> PRT
   <213> Homo sapiens
<400> 22
   Leu Glu Glu Met Gln Arg Arg Ala
   1 5
<210> 23
   <211> 7
   <212> PRT
   <213> Homo sapiens
<400> 23
   Leu Glu Glu Met Gln Arg Arg
   1 5
<210> 24
   <211> 10
   <212> PRT
   <213> Homo sapiens
<400> 24
   Glu Glu Met Gln Arg Arg Ala Asp Gln Leu
   1 5 10
<210> 25
   <211> 9
   <212> PRT
   <213> Homo sapiens
<400> 25
   Glu Glu Met Gln Arg Arg Ala Asp Gln
   1 5
<210> 26
   <211> 7
   <212> PRT
   <213> Homo sapiens
<400> 26
   Glu Glu Met Gln Arg Arg Ala
   1 5
<210> 27
   <211> 6
   <212> PRT
   <213> Homo sapiens
<400> 27
   Lys Asn Leu Thr Asp Leu
   1 5
<210> 28
   <211> 20̸
   <212> PRT
   <213> Homo sapiens
<400> 28
   Ser Asn Lys Thr Arg Ile Asp Glu Ala Asn Gln Arg Ala Thr Lys Met
   1 5 10 15
   Leu Gly Ser Gly
   20
<210> 29
   <211> 17
   <212> PRT
   <213> Homo sapiens
<400> 29
   Thr Arg Ile Asp Glu Ala Asn Gln Arg Ala Thr Lys Met Leu Gly Ser
   1               5                   10                  15
   Gly
<210> 30
   <211> 14
   <212> PRT
   <213> Homo sapiens
<400> 30
   Asp Glu Ala Asn Gln Arg Ala Thr Lys Met Leu Gly Ser Gly
   1               5                   10
<210> 31
   <211> 11
   <212> PRT
   <213> Homo sapiens
<400> 31
   Asn Gln Arg Ala Thr Lys Met Leu Gly Ser Gly
   1               5                   10
<210> 32
   <211> 10
   <212> PRT
   <213> Homo sapiens
<400> 32
   Gln Arg Ala Thr Lys Met Leu Gly Ser Gly
   1               5                   10

## Description

### FIELD OF THE INVENTION

The present invention refers to a composition for the treatment of pain and/or inflammation, preferably for the treatment of acute pain, chronic pain, inflammatory pain, pain induced by cancer or by cancer treatment, visceral pain, neuropathic pain, post-herpetic neuralgia, diabetic neuropathy, trigeminal neuralgia, migraine and fibromyalgia. This composition contains an effective amount of at least one peptide that possesses a sequence derived from the amino acid sequence of the SNAP-25 protein, or of its cosmetically or pharmaceutically acceptable salts.

### BACKGROUND OF THE INVENTION

Pain represents a serious social and economic problem. It is calculated that more than two million people are incapacitated on a daily basis from suffering transitory or chronic painful sensations *[*Williams, M., Kowaluk, E.A. and Arneric S.P (1999) "Emerging Molecular approaches to pain therapy" J. Med. Chem. 42, 1481-1500*].* Clear examples are algesia experienced by patients suffering from cancer, migraine, arthritis, burns, accidents and surgery. Pain that is not effectively treated can be devastating for people, limiting their capabilities, reducing their mobility, causing sleep disorders and dramatically interfering with their quality of life. Despite the seriousness of the problem, the pharmaceutical arsenal to combat, prevent and/or minimize its symptoms and its progress is surprisingly limited, partly due to the lack of specific therapeutic targets on which to act, and ignorance of the metabolic pathways that mediate the transduction of pain.

The integrity of our body is ensured by the proper, coupled operation of two highly specialized systems: the immune system and the nervous system. In the case of a tissue injury caused by noxious stimuli of a physical or chemical nature, both systems work in concert to cause sensitization of the affected area, with the objective of halting the spread of the damage and ensuring the speedy repair of the damaged area *[*Belmonte, C. and Cervero, F. Eds. (1996) "Neurobiology of Nociceptors" Oxford University Press*].* This process is called inflammation and can be of one of two types, either humoral if it is primarily mediated by the immune system, or neurogenic if it is caused by the nervous system. Either way, one important aspect is that both types of inflammation enhance each other, thus intensifying the experience of pain that accompanies the inflammatory process.

The sensation of pain begins when the peripheral terminals of a group of sensorial neurons, known as nociceptor neurons, or nociceptors, are activated by chemical, mechanical or thermal noxious stimuli. Nociceptor neurons convey information about tissue damage to the processing centers of the sensation of pain in the spinal cord and the brain *[*Belmonte, C. and Cervero, F. Eds. (1996) "Neurobiology of Nociceptors" Oxford University Press*;* Baranauskas, G. and Nistri, A. (1998) "Sensitization of pain pathways in the spinal cord: cellular mechanisms" Prog. Neurobiol. 54, 349-365; Richardson, D.J. and Vasko, M.R. (2002) "Cellular mechanisms of neurogenic inflammation" J. Pharmacol. Exp. Ther. 302, 839-845*]*. An important characteristic of nociceptors is that although they are mainly primary afferent neurons, once activated, they are capable of exerting an efferent function by releasing pro-algesic and pro-inflammatory molecules as substance P (SP), the peptide related to calcitonin (CGRP), histamine, ATP, glutamate, and bradykinin (BK). These molecules promote autocrine and paracrine activation of neighboring neurons as well as other cell types such as mast cells, neutrophils and platelets. When activated, the surrounding non-neuronal cells release neurotrophins (NGF), cytokines (a-TNF, IL1-β, IL-6), prostaglandins, leukotrienes and protons that give the inflammatory soup its acidic nature. All these factors, in turn, act on nociceptors enhancing local inflammation or neurogenic inflammation, altering nociceptive excitability or peripheral sensitization, and causing changes in the perception of stimuli applied to the damaged area, such as hyperalgesia, defined as an exaggerated response to a modestly harmful stimulus, such as mild temperatures of 35-40°C, or allodynia, defined as the phenomenon in which stimuli that are not harmful are perceived as painful, such as a light breeze *[*Belmonte, C. and Cervero, F. Eds. (1996) "Neurobiology of Nociceptors" Oxford University Press*;* Baranauskas, G. and Nistri, A. (1998) "Sensitization of pain pathways in the spinal cord: cellular mechanisms" Prog. Neurobiol. 54, 349-365; Richardson, D.J. and Vasko, M.R. (2002) "Cellular mechanisms of neurogenic inflammation" J. Pharmacol. Exp. Ther. 302, 839-845; Brune, K. and Handwerker, H.O. Eds. (2004) "Hyperalgesia: molecular mechanisms and clinical implications" Progress in Pain Research and Management, vol 30. IASP Press. Seatt*le*]*.* Persistent excitability of peripheral nociceptors causes synaptic changes at the level of the spinal cord leading to a central sensitization process, which in turn, helps to enhance pain perception in the inflamed area *[*Richardson, D.J. and Vasko, M.R. (2002) "Cellular mechanisms of neurogenic inflammation" J. Pharmacol. Exp. Ther. 302, 839-845; Brune, K. and Handwerker, H.O. Eds. (2004) "Hyperalgesia: molecular mechanisms and clinical implications" Progress in Pain Research and Management, vol 30. IASP Press. Seatt*le].*

The molecular and cellular bases of neurogenic inflammation and their regulation by inflammation mediators are largely unknown, mainly due to the lack of information about the molecular identity of many of the receptors involved and the uncertainty of the signaling pathways involved in nociceptors. However, it is known that the mechanisms by which the components of the inflammatory soup alter neuronal excitability can occur directly through interaction with ion channels on the surface of nociceptors or indirectly through intracellular cascades *[*Richardson, D.J. and Vasko, M.R. (2002) "Cellular mechanisms of neurogenic inflammation" J. Pharmacol. Exp. Ther. 302, 839-845; Brune, K. and Handwerker, H.O. Eds. (2004) "Hyperalgesia: molecular mechanisms and clinical implications" Progress in Pain Research and Management, vol 30. IASP Press. Seatt*le].* Thus the BK, the NGF and the interleukins produce their actions by activating metabolic pathways that activate PKC and PKA quinase proteins which can alter the membrane receptors that transduce environmental stimuli and/or modulate the expression of neuronal genes (especially in chronic inflammatory processes). The target receptors of the intracellular signaling pathways include channels activated by selective voltage to the Na⁺ ion and the receptor of TRPV1 vanilloids, a sensory integrator of chemical and thermal noxious stimuli, as well as mechanosensitive channels. The activation of these receptors triggers action potentials that stimulate the afferent and efferent function of the nociceptors, resulting in an increase in peripheral and central sensitization. Therefore, all these observations demonstrate a key role in the neurogenic or neurologic inflammation in acute and chronic inflammatory processes. Consequently, compounds that reduce the magnitude of neurogenic inflammation will present an anti-inflammatory and analgesic activity. So, for example, implicated neuronal receptor antagonists, such as TRPV1, Na⁺ channels, bradykinin receptors or purinegenic receptors will behave as powerful anti-inflammatory and/or analgesics agents. Evidences of this are the antagonists of the TRPV1 receptor *[*Garcia-Martinez, C., Planells-Cases, R., Fernandez, A.M. Royo, M., Albericio, F., Messeguer, A., Pérez-Payá, E., Carreño, C. and Ferrer-Montiel, A. (2003) "Small molecules targeting the TRPV1 complex as new drugs for pain management" Drugs of the Future 28, 15-23*].*

Despite this knowledge, current anti-inflammatory and/or analgesic compounds are limited to non-steroidal anti-inflammatory drugs (NSAIDs) such as aspirin or ibuprofen and narcotics such as morphine. NSAIDs have side effects that limit their usefulness; on the one hand, they have a ceiling of activity over which an increase in the dose does not decrease pain, on the other hand, they can also cause irritation in the intestinal tract, and therefore their prolonged use can lead to the development of a gastric ulcer. This is truly critical in elderly patients, who frequently consume NSAIDs on a daily basis for the treatment of chronic arthritic pathologies. Unfortunately, opioids also have unwanted side effects, such as constipation, respiratory system depression and psychoactive effects such as euphoria, sedation and addiction. These side effects occur at doses similar to those used in treatment, so that the doses that can be administered to patients are severely limited, meaning that their use is often relegated to the treatment of terminal patients.

There is thus a significant need to increase the existing pharmacological arsenal for treating pain.

However, in addition to action at the level of neuronal receptors, the molecular bases of neurogenic inflammation also involve an additional therapeutic target, such as blocking or inhibiting the release of pro-inflammatory (or pro-algesics) neural substances like CGRP, substance P, L-glutamate, ATP, histamine, etc., which are responsible for stimulating the immune and nervous systems. Pro-algesic neuronal substances are released through a mechanism of exocytosis dependent on cation Ca²⁺ and mediated by SNARE proteins *[*Bennett, M.K. and Scheller, R.H. (1993) "The molecular machinery for secretion is conserved from yeast to neurons" Proc. Natl. Acad. Sci. USA 90, 2559-2563*;* Südhof, T.C. (1995) "The synaptic vesicle cycle: a cascade of protein-protein interactions" Nature 375, 645-653; Yang, Y., Xia, Z., and Liu, Y. (2000) "SNAP25 functional domains in SNARE core complex assembly and glutamate release of cerebellar granule cells" J. Biol. Chem. 275, 29482-29487; Brunger, A.T. (2001) "Structure of proteins involved in synaptic vesicle fusion in neurons" Annu. Rev. Biophys. Biolmol. Struct. 30, 157-171*;* Chen, Y.A. and Scheller, R.H. (2001) "SNARE-mediated membrane fusion" Nat. Rev. Mol. Cell Biol. 2, 98-106*].*

It is known in the state of the art that the subcutaneous injection of botulinum toxin A, a potent inhibitor of neuronal exocytosis, which destroys the SNAP-25 protein *[*Bennett, M.K. and Scheller, R.H. (1993) "The molecular machinery for secretion is conserved from yeast to neurons" Proc. Natl. Acad. Sci. USA 90, 2559-2563; Südhof, T.C. (1995) "The synaptic vesicle cycle: a cascade of protein-protein interactions" Nature 375, 645-653*]*, reduces the pain produced by intraplantar administration of the chemical irritant formalin *[*Cui, M., Khanijou, S., Rubino, J. and Aoki, K.R. (2004) "Subcutaneous administration of botulinum toxin A reduces formalin-induced pain" Pain 107, 125-133*].*

The development of different serotypes of botulinum toxin for treating various types of pain is known in the state-of-the-art, as, for example and not limited thereto, the therapeutic applications described in patents US 7,381,700, US 7,374,769, US 7,361,358, US 7,294,339, US 7,255,866, US 7,211,262, US 7,172,763, US 7,091,176, US 7,067,137, US 6,887,476, US 6,869,610, US 6,838,434, US 6,776,992, US 6,641,820, US 6,623,742, US 6,565,870, US 6,500,436, US 6,464,986, US 6,458,365, US 6,423,319, US 6,372,226, US 6,333,037, US 6,235,289, US 6,113,915 and US 5,714,468 *inter alia.*

However, the inherent toxicity of botulinum toxin causes its administration, in a broad range of doses, to involve unwanted side effects, such as immunogenic responses, headaches, nausea, muscle paralysis or weakness, respiratory failure and, in the most extreme cases, the death of the treated subject *[*FDA News, February 8, 2008, "FDA Notifies Public of Adverse Reactions Linked to Botox Use*";* Coté, T.R., Mohan, A.K., Polder, J.A., Walton, M.K. and Braun, M.M. (2005) "Botulinum toxin type A injections: Adverse events reported to the US Food and Drug Administration in therapeutic and cosmetic cases" J. Amer. Acad. Derm. 53 (3), 407-415*].* These severe side effects, together with the high cost of treatment, severely limit the application of botulinum toxin for treating pain and/or inflammation, relegating it to chronic applications and/or pathologies for which there is no proper treatment. Therefore, there is a need to find safer alternative treatments with compounds that mimic the action of botulinum toxin but do not induce immune reactions, have fewer side effects and whose production cost is cheaper.

The applicant of this invention has determined that there are compounds which may show anti-inflammatory and/or analgesic activity by interfering with the formation of the SNARE complex required for neuronal exocytosis and solve the problems presented by treatment with botulinum toxin. It is known in the state of the art that certain peptides derived from the sequences of proteins that form the SNARE complex can inhibit neuronal exocytosis, such as, for example, peptides derived from the amino and carboxyl domains of the SNAP-25 protein *[*Apland, J.P., Biser, J.A., Adler, M., Ferrer-Montiel, A.V., Montal, M., Canaves, J.M., and Filbert, M.G. (1999) "Peptides that mimic the carboxy-terminal domain of SNAP-25 block acetylcholine release at an aplysia synapse" J. Appl. Toxicol. 19, Suppl.1: S23-S26*;* Mehta, P.P., Batternger, E., and Wilson, M. (1996) "SNAP-25 and synaptotagmin involvement in the final Ca2+-dependent triggering of neurotransmitter exocytosis" Proc. Natl. Acad. Sci. USA 93: 10471-10476*;* Ferrer-Montiel, A.V., Gutierrez, L.M., Apland, J.P., Canaves, J.M., Gil, A., Viniegra, S., Biser, J.A., Adler, M., and Montal, M. (1998) "The 26-mer peptide released from cleavage by botulinum neurotoxin E inhibits vesicle docking" FEBS Lett. 435, 84-88*;* Gutierrez, L.M., Canaves, J.M., Ferrer-Montiel, A.V., Reig, J.A., Montal, M. and Viniegra, S. (1995) "A peptide that mimics the carboxy-terminal domain of SNAP-25 blocks Ca2+-dependent exocytosis in chromaffin cells" FEBS Lett. 372, 39-43*;* Gutierrez, L.M., Viniegra, S., Rueda, J., Ferrer-Montiel, A.V., Canaves, J.M., and Montal, M. (1997) "A peptide that mimics the C-terminal sequence of SNAP-25 inhibits secretory vesicle docking in chromaffin cells" J. Biol. Chem. 272, 2634-2639*;* Blanes-Mira, C, Valera, E., Fernández-Ballester, G., Merino, J.M., Viniegra, S., Gutierrez, L.M., Perez-Paya, E., and Ferrer-Montiel, A. (2004) "Small peptides patterned after the N-terminus domain of SNAP-25 inhibit SNARE complex assembly and regulated exocytosis" J. Neurochem. 88, 124-135*],* peptides derived from the amino acid sequence of syntaxin *[*Martin, F., Salinas, E., Vazquez, J., Soria, B., and Reig, J.A. (1996) "Inhibition of insulin release by synthetic peptides show that the H3 region at the C-terminal domain of syntaxin-1 is crucial for Ca2+-but not for guanosine 5'-[gamma-thio]triphosphate-induced secretion" Biochem. J. 320, 201-205*],* of synaptobrevin *[*Cornille, F., Deloye, F., Fournie-Zaluski, M.C., Roques, B.P. and Poulain, B. (1995) "Inhibition of neurotransmitter release by synthetic proline-rich peptides shows that the N-terminal domain of vesicle-associated membrane protein/synaptobrevin is critical for neuro-exocytosis" J. Biol. Chem. 270, 16826-16830*],* of synaptotagmin *[*Mehta, P.P., Batternger, E., and Wilson, M. (1996) "SNAP-25 and synaptotagmin involvement in the final Ca2+-dependent triggering of neurotransmitter exocytosis" Proc. Natl. Acad. Sci. USA 93: 10471-104*]* and of the Snapin protein *[*llardi, J.M., Mochida, S., and Sheng, Z.H. (1999) "Snapin: A SNARE associated protein implicated in synaptic transmission" Nat. Neurosci. 2, 119-124*].* Similarly, synthetic peptides have also been obtained by rational design or by scanning synthetic chemical libraries, which can interfere with the formation of the SNARE complex by inhibiting neuronal exocytosis *[*Blanes-Mira, C., Pastor, M.T., Valera, E., Fernandez-Ballester, G., Merino, J.M., Gutierrez, L.M., Perez-Paya, E., and Ferrer-Montiel, A. (2003) "Identification of SNARE complex modulators that inhibit exocytosis form an α-helix-constrained combinatorial library" Biochem J. 375, 159-166*].*

The industrial application of this type of compounds has been limited. The cosmetic industry has made significant efforts to develop compounds that mimic the action of botulinum toxins for exclusive use in the treatment and prevention of the formation of expression wrinkles *[*Blanes-Mira, C., Clemente, J., Jodas, G., Gil, A., Fernandez-Ballester, G., Ponsati, B., Gutierrez, L.M., Pérez-Payá, E. and Ferrer-Montiel, A. (2002) "A synthetic hexapeptide (Argireline®) with anti-wrinkle activity" Int. J. Cosmet. Sci. 24, 303-310*].* Specifically, the patent applications EP1180524 A1 and WO2008/049945 of Lipotec, S.A. describe peptides derived from the amino terminal fragment of the SNAP-25 protein, free or modified at their amino and/or carboxy-terminal ends which possess anti-wrinkle effect, and the international application WO97/34620 also describes peptides derived from the amino acid sequence of the SNAP-25 protein, in particular its carboxy-terminal, or of synaptobrevin or syntaxin capable of inhibiting neuronal exocytosis.

None of the patents described above refers to the use of peptides derived from the SNAP-25 protein as an analgesic and/or anti-inflammatory agent, nor, in particular, the use of peptides derived from the SNAP-25 protein for the treatment of pain and/or inflammation.

This invention provides a solution to existing needs, which comprises the demonstration that peptides derived from the SNAP-25 protein, which block neuronal exocytosis, are anti-inflammatory and/or analgesic.

### DESCRIPTION OF THE INVENTION

This invention provides a simple, effective and risk-free solution for the treatment of pain and/or inflammation, which comprises the application of a composition which contains at least one peptide as claimed in claim 1.

Consequently, a first aspect of this invention concerns a composition for use in the treatment of pain and/or inflammation, which comprises an effective amount of at least one peptide according to the general formula (I):

R₁-AA-R₂ (I)

its stereoisomers and mixtures thereof, racemic or not, and its cosmetically or pharmaceutically acceptable salts thereof,
wherein AA is a sequence of adjacent amino acids selected from the group consisting of SEQ ID No.4, SEQ ID No.8, SEQ ID No.9, SEQ ID No.11, SEQ ID No.14, SEQ ID No. 15, SEQ ID No.16, SEQ ID No.17, SEQ ID No.18, SEQ ID No.19, SEQ ID No.20, SEQ ID No.21, SEQ ID No.22, SEQ ID No.23, SEQ ID No.24, SEQ ID No.25 and SEQ ID No.26; R₁ is selected from the group consisting of H, a polyethylene glycol polymer 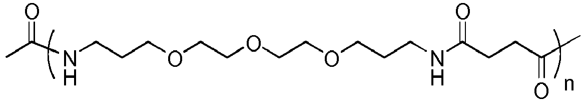 wherein n can range between 1 and 5, and R₅ -C(O)-, wherein R₅ is substituted or non-substituted non-cyclic aliphatic group of C₁ to C₂₄, or a substituted or non-substituted alicyclyl group of C₁ to C₂₄; and R₂ is selected from the group consisting of -NR₃R₄ and -OR₃, wherein R₃ and R₄ are selected independently from the group consisting of H, substituted or non-substituted non-cyclic aliphatic group of C₁ to C₂₄ and substituted or non-sustituted alicyclyl group of C₁ to C₂₄.

In one particular embodiment, the composition for the treatment of pain and/or inflammation is a cosmetic or pharmaceutical composition.

In another particular embodiment, preferred structures are those wherein R₁ is H, acetyl, *tert*-butanoyl, hexanoyl, 2-methylhexanoyl, cyclohexancarboxyl, octanoyl, decanoyl, lauroyl, miristoyl, palmitoyl, stearoyl, oleoyl and linoleoyl.

In another particular embodiment, preferred structures are those wherein R₃ and R₄ are selected independently from the group consisting of H, methyl, ethyl, hexyl, dodecyl and hexadecyl.

Peptides comprised in the composition of this invention may exist as stereoisomers or mixtures of stereoisomers; for example, the amino acids that make them up can have L-, D-configuration or be racemic independently from each other. Therefore, it is possible to obtain isomeric mixtures as well as racemates or diastereomeric mixtures or pure diastereomers or enantiomers, depending on the number of asymmetric carbons and what isomers or isomeric mixtures are present. The preferred structures of the peptides comprised in the composition of the invention are pure isomers, i.e., enantiomers or diastereomers.

Within the context of this invention, the term "non-cyclic aliphatic group" is used in this invention to encompass, for example and not limited thereto, alkyl, alkenyl and alkynyl groups, linear or branched.

The term "alkyl group" refers in this invention to a linear or branched saturated group, having 1 to 24, preferably 1 to 16, even more preferably 1 to 14, still more preferably 1 to 12, still more preferably 1, 2, 3, 4, 5 or 6 carbon atoms, and which is bound to the rest of the molecule through a simple bond, including, for example and not limited thereto, methyl, ethyl, isopropyl, isobutyl, *tert*-butyl, heptyl, octyl, decyl, dodecyl, lauryl, hexadecyl, octadecyl, amyl, 2-ethylhexyl, 2-methylbutyl, 5-methylhexyl and the like.

The term "alkenyl group" refers in this invention to a group that has between 2 and 24, preferably between 2 and 16, even more preferably between 2 and 14, still more preferably between 2 and 12, still more preferably 2, 3, 4, 5 or 6 carbon atoms with one or more carbon-carbon double bonds, preferably with 1, 2 or 3 carbon-carbon double bonds, conjugated or not conjugated, which is bound to the rest of the molecule through a simple bond, including for example and not limited thereto, the vinyl, oleyl, linoleyl group and the like.

The term "alkynyl group" refers in this invention to a group that has between 2 and 24, preferably between 2 and 16, even more preferably between 2 and 14, still more preferably between 2 and 12, still more preferably 2, 3, 4, 5 or 6 carbon atoms with one or more triple carbon-carbon bonds, preferably 1, 2 or 3 triple carbon-carbon bonds, conjugated or not conjugated, which is bound to the rest of the molecule through a simple bond, including, for example and not limited thereto, the ethynyl group, 1-propynyl, 2-propynyl, 1-butynyl, 2-butynyl, 3-butinyl, pentynyl, e.g. 1-pentynyl, and the like.

The term "alicyclyl group" is used in this invention to encompass, for example, and not limited thereto, cycloalkyl or cycloalkenyl or cycloalkynyl groups.

The term "cycloalkyl" refers in this invention to a mono- or polycyclic saturated aliphatic group which has between 3 and 24, preferably between 3 and 16, even more preferably between 3 and 14, still more preferably between 3 and 12, and still more preferably 3, 4, 5 or 6 carbon atoms and which is bound to the rest of the molecule through a simple bond, including, for example and not limited thereto, cyclopropyl, cyclobutyl, cyclopentyl, cyclohexyl, cycloheptyl, methyl cyclohexyl, dimethyl cyclohexyl, octahydroindene, decahydronaphtalene, dodecahydro phenalene and the like.

The term "cycloalkenyl" refers in this invention to a mono- or polycyclic non-aromatic aliphatic group that has between 5 and 24, preferably between 5 and 16, even more preferably between 5 and 14, still more preferably between 5 and 12, and still more preferably 5 or 6 carbon atoms with one or more carbon-carbon double bonds, preferably 1, 2 or 3 carbon-carbon double bonds, conjugated or not conjugated, and which is bound to the rest of the molecule through a simple bond, including for example and not limited thereto, the cyclopent-1-en-1-yl group and the like.

The term "cycloalkynyl" refers in this invention to a mono- or polycyclic non-aromatic aliphatic group that has between 5 and 24, preferably between 5 and 16, even more preferably between 5 and 14, still more preferably between 5 and 12, and still more preferably 5 or 6 carbon atoms with one or more carbon-carbon double bonds, preferably 1, 2 or 3 carbon-carbon triple bonds, conjugated or not conjugated, and which is bound to the rest of the molecule through a simple bond, including for example and not limited thereto, the cyclohex-1-in-1-yl group and the like.

The term "aryl group" refers in this invention to an aromatic group which has between 6 to 30, preferably between 6 and 18, even more preferably between 6 and 10, and still more preferably 6 or 10 carbon atoms, composed of 1 2, 3 or 4 aromatic rings linked through a carbon-carbon bond or fused, including, for example and not limited thereto, phenyl, naphthyl, diphenyl, indenyl, phenanthryl or anthranyl *inter alia,* or an aralkyl group.

The term "aralkyl group" refers in this invention to an alkyl group substituted with an aromatic group, having between 7 and 24 carbon atoms and including, for example and not limited thereto, -(CH₂)₁₋₆-phenyl, -(CH₂)₁₋₆-(1-naphthyl), -(CH₂)₁₋₆-(2-naphthyl),-(CH₂)₁₋₆-CH(phenyl)₂ and the like.

The term "heterocyclyl group" refers in this invention to a hydrocarbon ring with 3-10 members in which one or more of the ring atoms, preferably 1, 2 or 3 ring atoms is an element different from carbon such as for example nitrogen, oxygen or sulfur and which may be saturated or unsaturated. For the purposes of this invention, the heterocycle can be a cyclic, mono-cyclic, bicyclic or tricyclic system, which may include fused ring systems, and atoms of nitrogen, carbon or sulfur may optionally be oxidized in the heterocyclyl radical; the nitrogen atom can be optionally quaternized and the radical heterocyclic can be partially or completely saturated or be aromatic. More preferably, the term heterocyclic refers to a ring with 5 or 6 members.

The term "heteroarylalkyl group" refers in this invention to an alkyl group substituted with a substituted or non-substituted aromatic heterocyclyl group, the alkyl group having 1 to 3 carbon atoms and the aromatic heterocyclyl group between 2 and 24 carbon atoms and from 1 to 3 atoms other than carbon, including, for example and not limited thereto, -(CH₂)₁₋₆-imidazolyl, -(CH₂)₁₋₆-triazolyl, -(CH₂)₁₋₆-thienyl, -(CH₂)₁₋₆-furyl,-(CH₂)₁₋₆-pyrrolidinyl and the like.

As it is understood in this technical area, there may be a certain degree of substitution of the radicals defined above. Thus, there may be a substitution in any of the groups of this invention. References in this document to substituted groups in the groups of this invention indicate that the specified radical may be substituted in one or more available positions by one or more substituents, preferably in 1, 2 or 3 positions, more preferably in 1 or 2 positions, and even more preferably in 1 position. Such substituents include, for example, and not limited thereto, C₁-C₄ alkyl; hydroxyl; C₁-C₄ alkoxyl; amino; C₁-C₄ aminoalkyl; C₁-C₄ carbonyloxyl; C₁-C₄ oxycarbonyl; halogen such as fluorine, chlorine, bromine and iodine, cyano, nitro; azido; C₁-C₄ alkylsulfonyl; thiol; C₁-C₄ alkylthio; aryloxyl such as phenoxyl, -NR_{b} (C=NR_{b}) NR_{b}R_{c}; wherein R_{b} and R_{c} are independently selected from the group consisting of H, C₁-C₄ alkyl, C₂-C₄ alkenyl, C₂-C₄ alkynyl, C₃-C₁₀ cycloalkyl, C₆-C₁₈ aryl, C₇-C₁₇ aralkyl, heterocyclic with 3-10 members or amino protective group.

In the context of this invention "amino acid sequence derived from the amino acid sequence from the SNAP-25 protein" means any amino acid sequence or fragments of the amino acid sequence of the SNAP-25 protein, defined by the SEQ ID No.1, or any amino acid sequence that differs from the sequence SEQ ID No.1 by mutation, insertion, deletion or substitution of at least one amino acid, or by degeneration of the genetic code, provided that it corresponds to a peptide that possesses the activity of the SNAP-25 protein. Mutations, insertions or substitutions may take place by genetically encoded amino acids or non-coded amino acids, natural or not, for example, and not limited thereto, citrulline, ornithine, sarcosine, desmosine, norvaline, 4-aminobutyric acid, 2-aminobutyric acid, 2-aminoisobutyric acid, 6-aminohexanoic acid, 1-naphtylalanine, 2-naphtylalanine, 2-aminobenzoic acid, 4-aminobenzoic acid, 4-chlorophenylalanine, 2,3-diaminopropionic acid, 2,4- diaminobutyric acid, cycloserine, carnitine, cystine, penicillamine, pyroglutamic acid, thienylalanine, hydroxyproline, *allo-*isoleucine, a*llo*-threonine, isonipecotic acid, isoserine, phenylglycine, statin, beta-alanine, norleucine, N-methylamino acids, beta- or gamma-amino acids *inter alia* and their derivatives. A list of non-natural amino acids can be found in the article "Unusual amino acids in peptide synthesis" by Roberts D.C. and Vellaccio F., in "The Peptides", Vol 5 (1983), Chapter VI, Gross, E. and Meienhofer, J., Eds., Academic Press, New York, USA or in the commercial catalogs of companies specialized in the sector, such as NeoMPS, Bachem, Novabiochem, Sigma-Aldrich, Peptides International, Advanced ChemTech, Chem-Impex, Maybridge Chemical, Chirotech Technology, Peninsula Laboratories or RSP Amino Acid Analogues, *inter alia.*

The peptides derived from the amino acid sequence of SNAP-25 defined by SEQ ID No.1 are the sequences selected from the group consisting of SEQ ID No.4, SEQ ID No.8, SEQ ID No.9, SEQ ID No.11, SEQ ID No.14, SEQ ID No. 15, SEQ ID No.16, SEQ ID No.17, SEQ ID No.18, SEQ ID No.19, SEQ ID No.20, SEQ ID No.21, SEQ ID No.22, SEQ ID No.23, SEQ ID No.24, SEQ ID No.25, SEQ ID No.26.

Furthermore, disclosed are also compositions that comprise peptides substantially homologous to the peptides derived from the amino acid sequence of the SNAP-25 protein, irreversibly chemically modified. "Substantially homologous peptides" means in this invention those amino acid sequences that are at least 60%, preferably 80% and more preferably 95% identical to any of the preceding sequences. The "percentage of identity" refers to the percentage of amino acids that are identical between two compared amino acid sequences, after an optimal alignment of these sequences, where this percentage is purely statistical and differences between the two amino acid sequences are randomly distributed along the sequence. The term "optimal alignment" means the alignment of the amino acid sequences resulting in a higher percentage of identity. The percentage of identity is calculated by determining the number of identical positions where an amino acid is identical in the two sequences compared, dividing the number of identical positions by the number of positions compared and multiplying the result by 100 to get the percentage of identity between the two sequences. Sequence comparisons between two amino acid sequences can be carried out manually or by software such as BLAST algorithm (Basic Local Alignment Search Tool), available online at the site http://www.ncbi.nlm.nih.gov/BLAST/.

Within the scope of this invention are also included the cosmetically or pharmaceutically acceptable salts of the peptides of the compositions of the invention. The term "cosmetically or pharmaceutically acceptable salts" in this invention means a salt generally recognized for use in animals and more particularly in humans, including the salts used to form base addition salts, either inorganic, such as, for example, and without limitation thereto, lithium, sodium, potassium, calcium, magnesium or aluminum, *inter alia,* or organic such as, for example and not limited thereto, ethylamine, diethylamine, ethylenediamine, ethanolamine, diethanolamine, arginine, lysine, histidine or piperazine *inter alia,* or acid addition salts, either organic such as for example and without limitation thereto, acetate, citrate, lactate, malonate, maleate, tartrate, fumarate, benzoate, aspartate, glutamate, succinate, oleate, trifluoroacetate, oxalate, pamoate or gluconate, *inter alia,* or inorganic, such as, for example, and not limited thereto, chloride, sulfate, borate or carbonate *inter alia.* The nature of the salt is not critical, provided that it is cosmetically or pharmaceutically acceptable. The cosmetically or pharmaceutically acceptable salts of the peptides of the compositions of the invention can be obtained by conventional methods, well known in the state of the art *[*Berge, S.M., Bighley, L.D., and Monkhouse, D.C. (1977) "Pharmaceutical Salts" J. Pharm. Sci 66:1-19*].*

Additionally, the peptides for use of the invention can undergo reversible chemical modifications to enhance their bioavailability and ease of crossing of the blood-brain barrier or the epithelial tissue.

The peptides comprised in the compositions can be administered by any means that produces contact of the peptides with their action site in the body of a mammal, preferably human beings. These compositions can be prepared by conventional methods known by persons skilled in the art *["*Harry's Cosmeticology", Eight [sic] edition (2000) Rieger M.M., ed., New York Chemical Pub., NY, US*; "*Remington: The Science and Practice of Pharmacy", Twentieth edition (2003) Genaro A.R., ed., Lippincott Williams & Wilkins, Philadelphia, US*].*

The peptides comprised in the compositions have variable solubility in water, depending on the nature of their sequences or the possible modifications in their amino- and/or carboxy-terminal that they have. Therefore, the peptides of this invention can be incorporated into compositions by means of an aqueous solution, and those that are not soluble in water can be solubilized in cosmetically or pharmaceutically acceptable conventional solvents such as, for example, and not limited thereto, ethanol, propanol, isopropanol, propylene glycol, glycerin, butylene glycol or polyethylene glycol or any combination thereof.

The effective amount of peptides comprised in the compositions, their stereoisomers, mixtures thereof or their cosmetically or pharmaceutically acceptable salts, which must be administered to treat pain and/or inflammation, as well as their dosage, will depend on a number of factors including age, patient condition, the cause of the pain and/or inflammation, the severity of the pain and/or inflammation, the route and frequency of administration and the particular nature of the peptides used.

"Effective amount" means a non-toxic but sufficient amount of at least one peptide to provide the desired effect. The peptides are used in the composition of this invention at concentrations effective to achieve the desired effect; preferably, in reference to the total weight of the composition, between 0.00000001% (by weight) and 20% (by weight), preferably between 0.000001% (by weight) and 20% (by weight), more preferably between 0.0001% (by weight) and 10% (by weight) and more specifically between 0.0001% (by weight) and 5% (by weight).

In another particular embodiment, the peptides comprised in the compositions can also be incorporated into delivery systems and/or sustained release systems.

The term "delivery systems" refers to a diluent, adjuvant, excipient or carrier with which the peptide derivative of the invention is administered. These carriers can be liquids such as water, oils and surfactants, including those of petroleum, animal, vegetable or synthetic origin, such as, for example, and not limited thereto, peanut oil, soybean oil, mineral oil, sesame oil, castor oils, polysorbates, sorbitan esters, ether sulfates, sulfates, betaines, glucosides, maltosides, fatty alcohols, nonoxynol, poloxamer, polyoxyethylenes, polyethylene glycols, dextrose, glycerol and the like. *"*Remington's Pharmaceutical Sciences" by E.W. Martin describes diluents, adjuvants or excipients as appropriate carriers.

The term "sustained release" is used in the conventional sense, referring to a delivery system for a compound that provides gradual release of said compound for a time period and preferably, though not necessarily, with constant release levels of the compound over a period of time.

Examples of delivery or sustained release systems are liposomes, milliparticles, microparticles, nanoparticles, sponges, vesicles, micelles, millispheres, microspheres and nanospheres, lipospheres, millicapsules, microcapsules, nanocapsules, microemulsions and nanoemulsions, which can be added to achieve greater penetration of the active ingredient and/or to improve its pharmacokinetic and pharmacodynamic properties.

In another particular embodiment, sustained release formulations can be prepared by methods known in the state of the art, and compositions containing them can be administered, for example, by topical administration, including adhesive patches and non-adhesive patches, or by systemic administration, such as, for example, and not limited thereto, by enteral or parenteral route and they preferably should release a relatively constant amount of the peptides comprised in the compositions of the invention. The amount of peptide contained in the sustained release formulation will depend, for example, on the site of administration, the kinetics and duration of the release of the peptide of the compositions of the invention, as well as the cause and severity of the pain and/or inflammation, route, frequency of administration and the particular nature of the peptides to be used.

In the context of this invention, the terms "enteral or parenteral" include oral, nasal, inhalational, rectal routes, adhesive or non-adhesive patches, subcutaneous, intradermal, intravascular injections, such as intravenous, intramuscular, intraarterial, intravitreal, spinal, intracranial, intraarticular, intrathecal and intraperitoneal, as well as any similar injection or infusion technique.

In another particular embodiment, the composition for use according to the invention additionally includes acceptable carriers and/or auxiliary agents necessary for the administration of the composition in the desired manner. Among the carriers and/or auxiliary agents are included excipients, thickeners, diluents, solvents, dispersants, agents to improve freeze-drying or adjuvants suitable for each route of administration and which are known to the man of the art. Thickeners include, but are not limited to, water-soluble polymers such as those selected from the group consisting of modified celluloses, methylcellulose, ethylcellulose, hydroxyethylcellulose, hydroxyethylmethylcellulose, hydroxypropylcellulose, hydroxypropylmethylcellulose and carboxymethylcellulose, dextrans, gelatins, collagen, hyaluronic acid, polyethylene glycol or polyvinyl pyrrolidone. Diluents and solvents include, but are not limited to, those selected from the group consisting of ethanol, polyethylene glycol, glycofurol, N-methyl-2-pyrrolidone, glycerol, propanediol, polypropylene glycol, benzyl alcohol or dimethylsulfoxide. Dispersants include, but are not limited to, surfactants selected from the group consisting of monoesters of fatty acids of polyoxyethylene sorbitan (Tween®, Emalex, Nikkol®, Hodag, Dacol or Liposorb®), fatty acid monoesters of sorbitan (Span®), 15-hydroxystearate polyethylene glycol (Solutol® HS15), fatty acid esters of polyethylene glycol (Crodet, Cithrol, Kessco®, Nikkol®, Mapeg®, Myrj, Tagat®, Aldo®, Capmul®, Glycerox, Lactomul® or Emerest®), esters of polyoxyethylene glycol (Emulphor®), polyethoxylated castor oils (Cremophor®, Emalex, Eumulgin®, Nikkol® or Simusol®), fatty acid esters of polyglycerol (Nikkol Decaglyn, Polymuls, Caprol®), polyethylene glycol ethers (Volpo or Brij®), poloxamers (Lutrol® or Pluronic®), phenyl ethers of polyoxyethylene (Triton® or Igepal®), or mixtures thereof. Agents to improve freeze-drying include, but are not limited to, sugars such as those selected from the group consisting of mannitol, saccharose, glucose, fructose, lactose, trehalose, sucrose, dextrose, sorbitol and glycine, gelatins, polyvinyl pyrrolidone, or mixtures thereof. Preferably, the composition for the treatment of pain and/or inflammation also comprises one or more acceptable excipients such as humectants, pH buffers, preservatives, bactericidal and fungicidal agents, absorption retardants, absorption accelerators, or any other excipient known to the man of the art.

In another particular embodiment, the peptides comprised in the compositions can also be adsorbed on solid organic polymers, or solid mineral carriers such as, but not limited to, talc, bentonite, silica, starch or maltodextrin, *inter alia.*

In another particular embodiment, the compositions for use according to the invention can also be incorporated into fabrics, non-woven fabrics and medical devices that are in direct contact with the skin, mucosae and/or the scalp, such that they release the peptides either by biodegradation of the anchoring system to the fabric, non-woven fabric or medical device or by the friction of these ones with the body, body moisture, the pH of the skin or by body temperature. Likewise, fabrics and non-woven fabrics can be used to make garments that are in direct contact with the body.

Examples of fabrics, non-woven fabrics, garments, medical devices and means of inmobilizing the peptides to them, including the delivery systems and/or sustained release systems described above can be found described in literature and are known in the state of the art *[*Schaab C.K. (1986) "Impregnating Fabrics With Microcapsules", HAPPI May 1986*;* Nelson G. (2002) "Application of microencapsulation in textiles" Int. J. Pharm. 242:55-62*; "*Biofunctional Textiles and the Skin" (2006) Curr. Probl. Dermatol. v.33, Hipler U.C. and Elsner P., eds. S. Karger AG, Basel, Switzerl*and;* Malcom R.K., McCullagh S.D., Woolfson A.D., Gorman S.P., Jones D.S. and Cuddy J. (2004) "Controlled release of a model antibacterial drug from a novel self-lubricating silicone biomaterial" J. Cont. Release 97:313-320*].* Preferred fabrics, non-woven fabrics, garments and medical devices are bandages, gauzes, T-shirts, socks, stockings, underwear, girdles, gloves, diapers, compresses, dressings, bedspreads, towelettes, hydrogels, adhesive patches, non-adhesive patches, micro-electric patches and/or facial masks.

In another particular embodiment, the compositions comprising the peptides, their stereoisomers, mixtures thereof or their cosmetically or pharmaceutically acceptable salts, can be used in different types of formulations for topical or transdermal application which will optionally contain the acceptable excipients necessary for the formulation of the desired dosage form *[*Fauli i Trillo C. (1993) in "Tratado de Farmacia Galénica"[Treatise on Galenic Pharmacy], Luzán 5, S.A. Ediciones, Madrid*].*

Formulations for topical or transdermal application can be presented in any solid, liquid or semi-solid dosage form, such as, for example, and not limited thereto, creams, multiple emulsions such as for example and not limited thereto, emulsions of oil and/or silicon in water, emulsions of water in oil and/or silicone, emulsions of water/oil/water or water/silicone/water and emulsions of oil/water/oil or silicone/water/silicone, anhydrous compositions, aqueous dispersions, oils, milks, balms, foams, lotions, gels, hydroalcoholic solutions, liniments, sera, soaps, shampoos, unguents, mousses, ointments, powders, bars, pencils and spray or aerosol ("sprays"), including "leave-on" formulations and "rinse-off" formulations. These formulations for topical or transdermal application can be incorporated by means of techniques known to the man of the art into different types of solid accessories such as, for example and not limited thereto, towelettes, hydrogels, adhesive patches, non-adhesive patches, or facial masks, or may be incorporated into different makeup line products.

In another particular embodiment, the compositions for use according to the invention can additionally include agents that enhance the percutaneous absorption of the peptides with general formula (I), their stereoisomers, mixtures thereof or their cosmetically or pharmaceutically acceptable salts, such as, for example, but not limited thereto, dimethylsulfoxide, dimethylacetamide, dimethylformamide, surfactants, azone (1-dodecylazacycloheptan-2-one), alcohol, acetone, propylene glycol or polyethylene glycol, *inter alia.* Likewise, the compositions of this invention can be applied to local areas to be treated through topical or transdermal route by intradermal injection, iontophoresis, sonophoresis, electroporation, mechanical pressure, osmotic pressure gradient, occlusive treatment, microinjections or pressure injections without needles, such as injections by pressure of oxygen, micro-electric patches, or any combination thereof, in order to achieve greater penetration of the peptide of the invention. The area of application will be determined by the nature of the pain and/or inflammation to treat.

The compositions for use according to the invention can also be administered, in addition to the topical or transdermal route, by any other appropriate means, e.g. by enteral or parenteral route, which will include the acceptable excipients necessary for formulation in the desired dosage form. A review of the different dosage forms of the active ingredients and excipients needed to obtain them can be found, for example, in the *"*Tratado de Farmacia Galénica", C. Fauli i Trillo, 1993, Luzán 5, S.A. Ediciones, Madrid*.*

In another particular embodiment, the composition for use according to the invention additionally comprises an effective amount of at least one active ingredient selected from the group consisting of an antioxidant agent, a NO-synthase inhibitor, a skin-relaxing agent, an anti-inflammatory agent, an analgesic agent, an antimicrobial agent, an antifungal agent, or mixtures thereof.

In another particular embodiment, this invention refers to a composition for use that contains an effective amount of at least one peptide with general formula (I), its stereoisomers, mixtures thereof or its cosmetically or pharmaceutically acceptable salts and an effective amount of at least one analgesic compound and/or anti-inflammatory compound for the purpose of enhancing the analgesic and/or anti-inflammatory effect of the compositions of the invention. Among these compounds are included synthetic compounds such as hydrocortisone, clobetasol, dexamethasone, prednisone, paracetamol, acetylsalicylic acid, amoxiprin, benorylate, choline salicylate, diflunisal, faislamine, methyl salicylate, magnesium salicylate, salsalate, diclofenac, aceclofenac, acemetacin, bromfenac, etodolac, indomethacin, sulindac, tolmetin, ibuprofen, carprofen, fenbufen, fenoprofen, flurbiprofen, ketoprofen, ketorolac, loxoprofen, naproxen, oxaprozin, tiaprofenic acid, suprofen, mefenamic acid, meclofenamate, meclofenamic acid, nabumetone, phenylbutazone, azapropazone, metamizole, oxifenbutazone, sulfinpyrazone, piroxicam, lornoxicam, meloxicam, tenoxicam, celecoxib, etoricoxib, lumiracoxib, parecoxib, rofecoxib, valdecoxib, nimesulide, licofelone, omega-3 fatty acids and their biometabolites, morphine, codeine, oxycodone, hydrocodone, diamorphine, pethidine, tramadol, brupenorphine, benzocaine, lidocaine, chloroprocaine, tetracaine, procaine, tricyclic antidepressants, amitriptyline, carbamazepine, gabapentin, pregabalin, bisabolol, panthenol, biotin, disodium lauriminodipropionate tocopheryl phosphate, ciclopiroxolamine, nordihydroguaiaretic acid, coenzyme Q10 or alkyl glycerol ethers, or natural extracts or essential oils with intrinsic analgesic and/or anti-inflammatory activity, such as, for example, but not limited thereto, madecassoside, echinacin, amaranth seed oil, sandalwood oil, placenta extract, peach leaf extract, *Aloe vera, Arnica montana, Artemisia vulgaris, Asarum maximum, Calendula officinalis, Capsicum, Centipede cunninghamii, Chamomilla recutita, Crinum asiaticum, Hamamelis virginiana, Harpagophytum procumbens, Hypericum perforatum, Lilium candidum, Malva sylvestris, Melaleuca alternifolia, Origanum majorana, Salix alba, Silybum marianum, Tanacetum parthenium* or *Uncaria guianensis, inter alia.*

The biological activity of the compositions of this invention was determined in animal models of pain and inflammation. The compositions of the invention can reduce the inflammation produced by intraplantar injection of carrageenan, as well as inhibit the thermal hyperalgesia produced by intraplantar injection of Complete Freund's Adjuvant (CFA).

In another particular embodiment, the compositions are suitable for the treatment of the pain and/or inflammation that occurs in response to various noxious stimuli (mechanical, chemical and thermal) that cause acute and chronic inflammatory pain, as well as from lesions in the nervous system that cause neuropathic pain, and pain and/or inflammation in those pathologies involving visceral pain. Among pain and inflammation are included, for example, but not limited thereto, neuropathic pain, inflammatory pain, visceral pain, including abdominal pain, pain of the digestive system, pain of the respiratory system, pain of the urogenital system, pain of the endocrine system, heart pain, pancreatic pain, intestinal pain, stomach pain, spleen pain, blood vessel pain, irritable bowel syndrome, tensional headache pain, headache associated with sinusitis, migraine, eye pain, dry eye syndrome, post-operative pain, including post-operative pain due to surgical incisions, the insertion of implants in bone, bone replacement and/or infection, pain due to cancer, including pain due to bone cancer, pain associated with benign bone tumors, including osteoid osteoma, osteoblastomas, pain due to cancer treatment, musculoskeletal pain, fibromyalgia, nerve pain, neck pain associated with cervical dystonia, back pain, including lumbago and/or sciatica, neurogenic inflammation, skin irritation, sensitive skin, atopic dermatitis, contact dermatitis, diaper dermatitis, eczema, arthritis, rheumatoid arthritis, osteoarthritis, post-herpetic neuralgia, peripheral neuropathies, phantom pain, allodynia, pain due to carpal tunnel syndrome, burning pain, paresthesias, facial pain, trigeminal neuralgia, neuropathic pain due to diabetes, pain associated with tattooing or tattoo removal, pain due to bunions, testicular pain, myofascial pain, spastic muscle pain, pain of the urinary bladder, pain of the urinary tract, vulvar pain, vaginal pain, scrotal pain, perineal pain, pelvic pain, pain or skin irritation after surgery, after treatment with pulsed light therapy (IPL, Intense Pulse Light), after treatment with pulsed monochromatic light therapy (laser), after treatment with chemical exfoliating agents or after overexposure to aggressive external agents such as overexposure to sunlight or extreme cold or heat.

In particular, the treatment of post-operative pain is done by administering the composition of the invention before, during or immediately after surgery. Preferably, the surgical procedure is selected from the group consisting of removal of tumors, bone implants, bone removal, cosmetic surgery procedures, exploratory surgery, and skin incisions.

A second aspect of this invention refers to a peptide with general formula (I),

R₁-AA-R₂ (I)

its stereoisomers and mixtures thereof, racemic or not, and its cosmetically or pharmaceutically acceptable salts, as defined in claim 1
for the treatment of pain and/or inflammation.

In another particular embodiment, preferred structures are those where R₁ is H, acetyl, *tert*-butanoyl, hexanoyl, 2-methylhexanoyl, cyclohexancarboxyl, octanoyl, decanoyl, lauroyl, miristoyl, Palmitoyl, stearoyl, oleoyl and linoleoyl.

In another particular embodiment, preferred structures are those where R₃ and R₄ are selected independently from the group consisting of H, methyl, ethyl, hexyl, dodecyl and hexadecyl.

Peptides used for the treatment of pain and/or inflammation may exist as stereoisomers or mixtures of stereoisomers; for example, the amino acids that make them up can have L- or D- configuration, or be racemic independently from one other. Therefore, it is possible to obtain isomeric mixtures as well as racemates or diastereomeric mixtures or pure diastereomers or enantiomers, depending on the number of asymmetric carbons and what isomers or isomeric mixtures are present. The preferred structures of the peptides are pure isomers, i.e., enantiomers or diastereomers.

Among the peptides derived from the sequence of amino acids of SNAP-25 defined by SEQ ID No.1 used to treat pain and/or inflammation, disclosed sequences are those that possess a sequence of adjacent amino acids contained in the sequence of the amino terminal region of the SNAP-25 protein, defined by SEQ ID No. 2 or the carboxy terminal region of the SNAP-25 protein, defined by SEQ ID No. 3, more preferably contained in the region between residues 10 to 22, defined by SEQ ID No.4, or contained in the region between residues 25 to 40, defined by SEQ ID No.5, or contained in the region between residues 65 to 81 defined by SEQ ID No.6, or contained in the region between residues 181 to 206, defined by SEQ ID No.7, more precisely in the region between residues 12 to 19, defined by the SEQ ID No.8, or contained in the region between residues 26 to 38, defined by SEQ ID No.9, or contained in the region between residues 68 to 79, defined by SEQ ID No.10, and specifically contained in the region between residues 12 to 17, defined by SEQ ID No.11.

In particular, preferred amino acid sequences are those that have a sequence of adjacent amino acids contained in any one of the sequences selected from the group consisting of SEQ ID No.4, SEQ ID No.5, SEQ ID No.6, SEQ ID No.7, SEQ ID No.8, SEQ ID No.9, SEQ ID No.10, SEQ ID No.11, SEQ ID No.12, SEQ ID No.13, SEQ ID No.14, SEQ ID No. 15, SEQ ID No.16, SEQ ID No.17, SEQ ID No.18, SEQ ID No.19, SEQ ID No.20, SEQ ID No.21, SEQ ID No.22, SEQ ID No.23, SEQ ID No.24, SEQ ID No.25, SEQ ID No.26, SEQ ID No.27, SEQ ID No.28, SEQ ID No.29, SEQ ID No.30, SEQ ID No.31 and SEQ ID No.32.

Within the scope of this invention are also included the cosmetically or pharmaceutically acceptable salts of peptides with general formula (I). The nature of the salt is not critical, provided that it is cosmetically or pharmaceutically acceptable. The cosmetically or pharmaceutically acceptable salts of the peptides can be obtained by conventional methods, well known in the state of the art *[*Berge S.M., Bighley L.D. and Monkhouse D.C. (1977) "Pharmaceutical Salts" J. Pharm. Sci. 66:1-19*].*

Additionally, peptides can undergo reversible chemical modifications to enhance their bioavailability and ease of crossing of the blood-brain barrier or the epithelial tissue.

Peptides with general formula (I), their stereoisomers, mixtures thereof, or their cosmetically or pharmaceutically acceptable salts for the treatment of pain and/or inflammation can be incorporated into compositions and can be administered by any means that produces contact of the peptides with their action site in the body of a mammal, preferably human beings. These compositions can be prepared by conventional methods known by persons skilled in the art *["*Harry's Cosmeticology", Eight [sic] edition (2000) Rieger M.M., ed., New York Chemical Pub., NY, US*; "*Remington: The Science and Practice of Pharmacy", Twentieth edition (2003) Genaro A.R., ed., Lippincott Williams & Wilkins, Philadelphia, US*].*

Peptides with general formula (I), their stereoisomers, mixtures thereof, or their cosmetically or pharmaceutically acceptable salts for the treatment of pain and/or inflammation have variable solubility in water, depending on the nature of their sequences or the possible modifications in their amino- and/or carboxy-terminal that they have. Therefore, the peptides can be incorporated into compositions by means of an aqueous solution, and those that are not soluble in water can be solubilized in cosmetically or pharmaceutically acceptable conventional solvents such as, for example and not limited thereto, ethanol, propanol, isopropanol, propylene glycol, glycerin, butylene glycol or polyethylene glycol or any combination thereof.

The effective amount of peptides with general formula (I), their stereoisomers, mixtures thereof, or their cosmetically or pharmaceutically acceptable salts which must be administered to treat pain and/or inflammation, as well as their dosage will depend on a number of factors, including age, patient condition, the cause of the pain and/or inflammation, the severity of the pain and/or inflammation, the route and frequency of administration and the particular nature of the peptides used.

Peptides with general formula (I), their stereoisomers, mixtures thereof, or their cosmetically or pharmaceutically acceptable salts are contained in the composition of concentrations effective to achieve the desired effect for the treatment of pain and/or inflammation; preferably, in reference to the total weight of the composition, between 0.00000001% (by weight) and 20% (by weight), more preferably between 0.000001% (by weight) and 20% (by weight), more preferably between 0.0001% (by weight) and 10% (by weight) and more specifically between 0.0001% (by weight) and 5% (by weight).

In another particular embodiment, the peptides with general formula (I), their stereoisomers, mixtures thereof, or their cosmetically or pharmaceutically acceptable salts for the treatment of pain and/or inflammation are incorporated into delivery systems and/or sustained release systems.

These carriers can be liquids such as water, oils or surfactants, including those of petroleum, animal, vegetable or synthetic origin, such as, for example, and not limited thereto, peanut oil, soybean oil, mineral oil, sesame oil, oil castor oils, polysorbates, sorbitan esters, ether sulfates, sulfates, betaines, glucosides, maltosides, fatty alcohols, nonoxynol, poloxamers, polyoxyethylenes, polyethylene glycols, dextrose, glycerol and the like. *"Remington's Pharmaceutical Sciences"* by E.W. Martin describes diluents, adjuvants or excipients as appropriate carriers.

Examples of delivery or sustained release systems are liposomes, milliparticles, microparticles, nanoparticles, sponges, vesicles, micelles, millispheres, microspheres and nanospheres, lipospheres, millicapsules, microcapsules, nanocapsules, microemulsions and nanoemulsions, which can be added to achieve greater penetration of the active ingredient and/or to improve its pharmacokinetic and pharmacodynamic properties.

In another particular embodiment, sustained release formulations can be prepared by methods known in the state of the art, and compositions containing them can be administered, for example, by topical administration, including adhesive patches and non-adhesive patches, or by systemic administration, such as, for example, and not limited thereto, by enteral or parenteral route and they preferably should release a relatively constant amount of the peptides of the composition. The amount of peptide contained in the sustained release formulation will depend, for example, on the site of administration, the kinetics and duration of the release of the peptide of the compositions of the invention, as well as the cause and severity of the pain and/or inflammation, the route, frequency of administration and the particular nature of the peptides to be used.

In another particular embodiment, the peptides with general formula (I), their stereoisomers, mixtures thereof, or their cosmetically or pharmaceutically acceptable salts for the treatment of pain and/or inflammation are incorporated into a composition which additionally includes acceptable carriers and/or auxiliary agents necessary for the administration of the composition in the desired manner. Among the carriers and/or auxiliary agents are included excipients, thickeners, diluents, solvents, dispersants, agents to improve freeze-drying or adjuvants suitable for each route of administration and which are known to the man of the art. Thickeners include, but are not limited to, water-soluble polymers such as those selected from the group consisting of modified celluloses, methylcellulose, ethylcellulose, hydroxyethylcellulose, hydroxyethylmethylcellulose, hydroxypropylcellulose, hydroxypropylmethylcellulose and carboxymethylcellulose, dextrans, gelatins, collagen, hyaluronic acid, polyethylene glycol or polyvinyl pyrrolidone. Diluents and solvents include, but are not limited to, those selected from the group consisting of ethanol, polyethylene glycol, glycofurol, N-methyl-2-pyrrolidone, glycerol, propanediol, polypropylene glycol, benzyl alcohol or dimethylsulfoxide. Dispersants include, but are not limited to, surfactants selected from the group consisting of monoesters of fatty acids of polyoxyethylene sorbitan (Tween®, Emalex, Nikkol®, Hodag, Dacol or Liposorb®), fatty acid monoesters of sorbitan (Span®), 15-hydroxystearate polyethylene glycol (Solutol® HS15), fatty acid esters of polyethylene glycol (Crodet, Cithrol, Kessco®, Nikkol®, Mapeg®, Myrj, Tagat®, Aldo®, Capmul®, Glycerox, Lactomul®, or Emerest®), esters of glycol polyoxyethylene (Emulphor®), polyethoxylated castor oils (Cremophor®, Emalex, Eumulgin®, Nikkol® or Simusol®), fatty acid esters of polyglycerol (Nikkol Decaglyn, Polymuls, Caprol®), polyethylene glycol ethers (Volpo or Brij®), poloxamer (Lutrol® or Pluronic®), phenyl ethers of polyoxyethylene (Triton® or Igepal®), or mixtures thereof. Agents to improve freeze-drying include, but are not limited to, sugars such as those selected from the group consisting of mannitol, saccharose, glucose, fructose, lactose, trehalose, sucrose, dextrose, sorbitol and glycine, gelatins, polyvinyl pyrrolidone, or mixtures thereof. Preferably, the composition that contains the peptide also contains one or more acceptable excipients such as humectants, pH buffers, preservatives, bactericidal and fungicidal agents, absorption retardants, absorption accelerators, or any other excipient known to the man of the art.

In another particular embodiment, the peptides with general formula (I), their stereoisomers, mixtures thereof, or their cosmetically or pharmaceutically acceptable salts for the treatment of pain and/or inflammation can also be adsorbed on solid organic polymers, or solid mineral carriers such as, but not limited to, talc, bentonite, silica, starch or maltodextrin, *inter alia.*

In another particular embodiment, the peptides with general formula (I), their stereoisomers, mixtures thereof, or their cosmetically or pharmaceutically acceptable salts are contained in compositions that can be incorporated into fabrics, non-woven fabrics and medical devices that are in direct contact with the skin, mucosae and/or the scalp, such that they release the peptides either by biodegradation of the anchoring system to the fabric, non-woven fabric or medical device or by the friction of these ones with the body, body moisture, the pH of the skin or body temperature. Likewise, fabrics and non-woven fabrics can be used to make garments that are in direct contact with the body.

Examples of fabrics, non-woven fabrics, garments, medical devices and means of inmobilizing of the peptides to them, including the delivery systems and/or sustained release systems described above can be found described in literature and are known in the state of the art *[*Schaab C.K. (1986) "Impregnating Fabrics With Microcapsules", HAPPI May 1986*;* Nelson G. (2002) "Application of microencapsulation in textiles" Int. J. Pharm. 242:55-62*; "*Biofunctional Textiles and the Skin" (2006) Curr. Probl. Dermatol. v.33, Hipler U.C. and Elsner P., eds. S. Karger AG, Basel, Switzerl*and;* Malcom R.K., McCullagh S.D., Woolfson A.D., Gorman S.P., Jones D.S. and Cuddy J. (2004) "Controlled release of a model antibacterial drug from a novel self-lubricating silicone biomaterial" J. Cont. Release 97:313-320*].* Preferred fabrics, non-woven fabrics, garments and medical devices are bandages, gauzes, T-shirts, socks, stockings, underwear, girdles, gloves, diapers, compresses, dressings, bedspreads, towelettes, hydrogels, adhesive patches, non-adhesive patches, micro-electric patches and/or facial masks.

In another particular embodiment, the peptides with general formula (I), their stereoisomers, mixtures thereof, or their cosmetically or pharmaceutically acceptable salts are contained in compositions that can be used in different types of formulations for topical or transdermal application which will optionally contain the acceptable excipients necessary for the formulation of the desired dosage form *[*Fauli i Trillo C. (1993) in "Tratado de Farmacia Galénica", Luzán 5, S.A. Ediciones, Madrid*].*

Formulations for topical or transdermal application can be presented in any solid, liquid or semi-solid dosage form, such as, for example, and not limited thereto, creams, multiple emulsions such as, for example and not limited thereto, emulsions of oil and/or silicon in water, emulsions of water in oil and/or silicone, emulsions of water/oil/water or water/silicone/water and emulsions of oil/water/oil or silicone/water/silicone, anhydrous compositions, aqueous dispersions, oils, milks, balms, foams, lotions, gels, hydroalcoholic solutions, liniments, sera, soaps, shampoos, unguents, mousses, ointments, powders, bars, pencils and sprays or aerosols ("sprays"), including "leave-on" formulations and "rinse-off" formulations. These formulations for topical or transdermal application can be incorporated by means of techniques known to the man of the art into different types of solid accessories such as, for example and not limited thereto, towelettes, hydrogels, adhesive patches, non-adhesive patches, or facial masks, or may be incorporated into different makeup line products.

In another particular embodiment, the peptides with general formula (I), their stereoisomers, mixtures thereof, or their cosmetically or pharmaceutically acceptable salts are contained in compositions that may include additional agents that enhance the percutaneous absorption of the peptides with general formula (I), their stereoisomers, mixtures thereof, or their cosmetically or pharmaceutically acceptable salts, such as, for example, and not limited thereto dimethylsulfoxide, dimethylacetamide, dimethylformamide, surfactants, azone (1-dodecylazacycloheptan-2-one), alcohol, acetone, propylene glycol or polyethylene glycol *inter alia.* In addition, these compositions can be applied to local areas to be treated through topical or transdermal route by intradermal injection, iontophoresis, sonophoresis, electroporation, mechanical pressure, osmotic pressure gradient, occlusive treatment, microinjections or injections without needles by pressure, such as injections by pressure of oxygen, micro-electric patches, or any combination thereof, in order to achieve greater penetration of the peptide of the invention. The area of application will be determined by the nature of pain and/or inflammation to treat.

Peptides with general formula (I), their stereoisomers, mixtures thereof, or cosmetically or pharmaceutically acceptable salts, which are contained in compositions can be administered, in addition, by topical or transdermal route, by any other appropriate means, for example by enteral or parenteral route, which include acceptable excipients necessary for formulation in the desired dosage form. A review of the different dosage forms of active ingredients and excipients to obtain them can be found, for example, in the "Tratado de Farmacia Galénica", C. Fauli i Trillo, 1993, Luzán 5, S.A. Ediciones, Madrid*.*

In another particular embodiment, peptides with general formula (I), their stereoisomers, mixtures thereof, or their cosmetically or pharmaceutically acceptable salts, are contained in compositions that additionally comprise an effective amount of at least one active ingredient selected from the group consisting of an antioxidant agent, a NO-synthase inhibitor, a skin relaxing agent, an anti-inflammatory agent, an analgesic agent, an antimicrobial agent, an antifungal agent, or mixtures thereof.

In another particular embodiment, peptides with general formula (I), their stereoisomers, mixtures thereof, or their cosmetically or pharmaceutically acceptable salts, are contained in compositions that also contain an effective amount of at least one analgesic compound and/or anti-inflammatory compound for the purpose of enhancing the analgesic and/or anti-inflammatory effect of the composition. Among these compounds can be highlighted synthetic compounds such as hydrocortisone, clobetasol, dexamethasone, prednisone, paracetamol, acetylsalicylic acid, amoxiprin, benorylate, choline salicylate, diflunisal, faislamine, methyl salicylate, magnesium salicylate, salsalate, diclofenac, aceclofenac, acemetacin, bromfenac, etodolac, indomethacin, sulindac, tolmetin, ibuprofen, carprofen, fenbufen, fenoprofen, flurbiprofen, ketoprofen, ketorolac, loxoprofen, naproxen, oxaprozin, tiaprofenic acid, suprofen, mefenamic acid, meclofenamate, meclofenamic acid, nabumetone, phenylbutazone, azapropazone, metamizole, oxifenbutazone, sulfinpyrazone, piroxicam, Lornoxicam, meloxicam, tenoxicam, celecoxib, etoricoxib, lumiracoxib, parecoxib, rofecoxib, valdecoxib, nimesulide, licofelone, omega-3 fatty acids and their biometabolites, morphine, codeine, oxycodone, hydrocodone, diamorphine, pethidine, tramadol, brupenorphine, benzocaine, lidocaine, chloroprocaine, tetracaine, procaine, tricyclic antidepressants, amitriptyline, carbamazepine, gabapentin, pregabalin, bisabolol, panthenol, biotin, disodium lauriminodipropionate tocopheryl phosphate, ciclopirox olamine, nordihydroguaiaretic acid, coenzyme Q10 or alkyl glycerol ethers, or natural extracts or essential oils with intrinsic analgesic and/or anti-inflammatory activity, for example, and not limited thereto madecassoside, echinacin, amaranth seed oil, sandalwood oil, placenta extract, peach leaf extract, *Aloe vera, Arnica montana, Artemisia vulgaris, Asarum maximum, Calendula officinalis, Capsicum, Centipede cunninghamii, Chamomilla recutita, Crinum asiaticum, Hamamelis virginiana, Harpagophytum procumbens, Hypericum perforatum, Lilium candidum, Malva sylvestris, Melaleuca alternifolia, Origanum majorana, Salix alba, Silybum marianum, Tanacetum parthenium* or *Uncaria guianensis, inter alia.*

The biological activity of peptides with general formula (I), their stereoisomers, mixtures thereof or their cosmetically or pharmaceutically acceptable salts, was established in animal models of pain and inflammation. These peptides are able to reduce the inflammation produced by intraplantar injection of carrageenan, as well as to inhibit the thermal hyperalgesia produced by intraplantar injection of Complete Freund's Adjuvant (CFA).

In another particular embodiment, peptides with general formula (I), their stereoisomers, mixtures thereof or their cosmetically or pharmaceutically acceptable salts are suitable for the treatment of the pain and/or the inflammation that occurs in response to various noxious stimuli (mechanical, chemical and thermal) that cause acute and chronic inflammatory pain, as well as from lesions in the nervous system that cause neuropathic pain, and pain and/or inflammation in those pathologies involving visceral pain. Among pain and inflammation are included, for example, and not limited thereto, neuropathic pain, inflammatory pain, visceral pain, including abdominal pain, pain of the digestive system, pain of the respiratory system, pain of the urogenital system, pain of the endocrine system, heart pain, pancreatic pain, intestinal pain, stomach pain, spleen pain, pain of the blood vessels, irritable bowel syndrome, tensional headache pain, headache associated with sinusitis, migraine, eye pain, dry eye syndrome, post-operative pain, including the post-operative pain due to surgical incisions, the insertion of implants in bone, the replacement of bone and/or to infection, pain due to cancer, including pain due to bone cancer , pain associated with benign bone tumors, including osteoid osteoma, osteoblastomas, pain due to cancer treatment, musculoskeletal pain, fibromyalgia, nerve pain, neck pain associated with cervical dystonia, back pain, including lumbago and/or sciatica, neurogenic inflammation , skin irritation, sensitive skin, atopic dermatitis, contact dermatitis, diaper dermatitis, eczema, arthritis, rheumatoid arthritis, osteoarthritis, post-herpetic neuralgia, peripheral neuropathies, phantom pain, allodynia, pain due to carpal tunnel syndrome, burning pain, paresthesias, facial pain, trigeminal neuralgia, neuropathic pain due to diabetes, pain associated with tattooing or tattoo removal, pain due to bunions, testicular pain, myofascial pain, spastic muscle pain, pain of the urinary bladder, pain of the urinary tract, vulvar pain, vaginal pain, scrotal pain, perineal pain, pelvic pain, pain or skin irritation after surgery, after treatment with pulsed light therapy (IPL, Intense Pulse Light), after treatment with pulsed monochromatic light therapy (laser), after treatment with chemical exfoliating agents or after overexposure to aggressive external agents such as overexposure to sunlight or extreme cold or heat.

In particular, the treatment of post-operative pain is done by administering an effective amount of the peptide of the composition of the invention before, during or immediately after surgery. Preferably, the surgical procedure is selected from the group consisting of removal of tumors, bone implants, bone removal, cosmetic surgery procedures, exploratory surgery, and skin incisions.

In another aspect, this invention provides the treatment of pain and/or inflammation, a method which comprises the administration of an effective amount of at least one peptide with general formula (I), its stereoisomers, mixtures thereof or its cosmetically or pharmaceutically acceptable salts, preferably in the form of a cosmetic or pharmaceutical composition that contains them. This invention also provides a method for the treatment of pain and/or inflammation comprising the application to the skin, mucosae and/or scalp, or enteral or parenteral administration of a composition containing at least one peptide with general formula (I), its stereoisomers, mixtures thereof or its cosmetically or pharmaceutically acceptable salts.

This invention also provides a method for the treatment and/or prevention of post-operative pain to a patient subundergone a surgical procedure which includes administering to said patient a therapeutically effective amount of at least one peptide with formula (I), its stereoisomers, mixtures thereof or cosmetically or pharmaceutically acceptable salts, preferably in the form of a pharmaceutical composition containing it, before, during or immediately after surgery. Preferably, the surgical procedure is selected from the group consisting of removal of tumors, bone implants, bone removal, cosmetic surgery procedures, exploratory surgery, and skin incisions.

### EXAMPLES

The following specific examples given here are intended to illustrate the nature of this invention. These examples are for illustrative purposes only and should not be construed as limitations on the invention herein claimed.

### General methodology

### Abbreviations:

The abbreviations used for amino acids follow the rules of the Commission on Biochemical Nomenclature of the IUPAC-IUB specified in Eur. J. Biochem. (1984) 138, 9-37 and J. Biol Chem (1989) 264, 633-673.

NSAIDs, non-steroidal anti-inflammatory drugs, ATP, adenosine triphosphate; BK, bradykinin; BoNT A, botulinum toxin serotype A; CFA, complete Freund's adjuvant; CGRP, calcitonin gene related peptide; IL, interleukin; NGF, neuronal growth factor; Palm, palmitoyl; PEG, polyethylene glycol; PEGₙ, -[NH-CH₂-(CH₂CH₂O)₃-(CH₂)₃-NH-CO-CH₂CH₂-CO-]ₙ; PKA protein kinase A, PKC, protein kinase C, SNAP-25, synaptosomal associated protein (25kDa), SP, substance P, TNF, tumor necrosis factor; TRPV1, transient receptor potential vanilloid 1.

### EXAMPLE 1. Peptides reduce inflammation produced by intraplantar injection of carrageenan.

To demonstrate that the peptides derived from the SNAP-25 protein have anti-inflammatory activity *in vivo* was used the carrageenan test. Carrageenan is an irritant whose administration causes a powerful inflammation four hours after administration. The inflammatory process can be easily discerned as an increase in the volume of the paw that received carrageenan, measured with a plethysmometer. Table 1 shows the values of anti-inflammatory activity of the peptides administered at 5 mg/kg (im) using diclofenac (10 mg/kg) as positive control and standardizing with respect to the values of the decrease in inflammation obtained by the positive control. Therefore, the peptides of this invention have anti-inflammatory activity *in vivo.*

**Table 1.**

| **COMPOSITION** | **ANTI-INFLAMMATORY ACTIVITY** |
|---|---|
| diclofenac | 100% |
| Ac-LESTRRMLQLVEE-NH₂ | 98% |
| Palm-EEMQRR-MH₂ | 81% |
| Palm-LESTRRMLQLVEE-NH₂ | 77% |
| Ac-ELEEMQRRADQLA-NH₂ | 65% |
| Palm-ELEEMQRRADQLA-NH₂ | 58% |
| Ac-PEG₅-EEMQRR-NH₂ | 53% |
| Ac-EEMQRR-NH₂ | 49% |
| Ac-PEG₃-EEMQRR-NH₂ | 42% |
| Ac-PEG₂-EEMQRR-NH₂ | 29% |
| Ac-PEG₄-EEMQRR-NH₂ | 23% |
| Ac-PEG₁-EEMQRR-NH₂ | 21% |
| Ac-EEMQRRA-NH₂ | 14% |

### EXAMPLE 2. Peptides inhibit the thermal hyperalgesia produced by intraplantar injection of Complete Freund's Adjuvant (CFA).

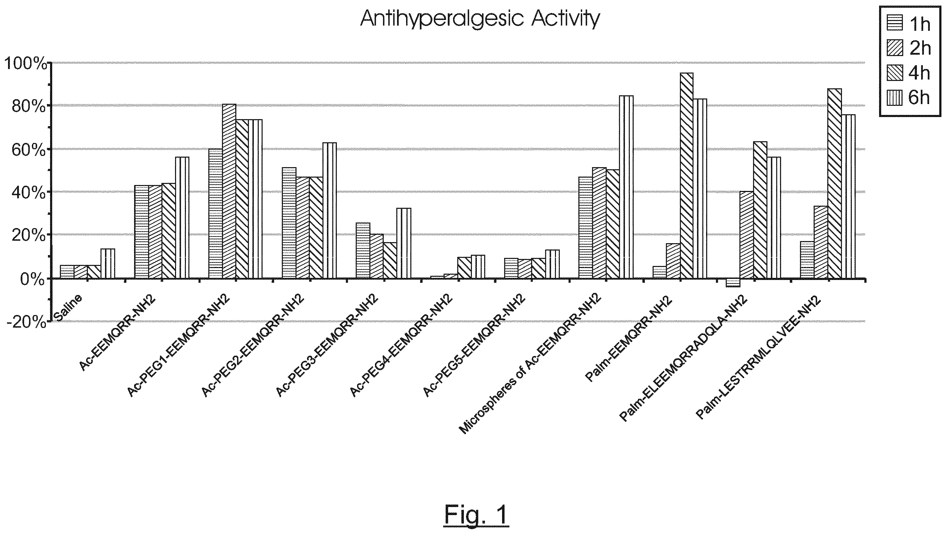

To evaluate the analgesic activity of the peptides in a model of chronic pain we used the intraplantar administration of CFA (1%) which produces an inflammatory process accompanied by thermal hyperalgesia at 24 hrs. after the administration of the irritant. Thermal hyperalgesia is easily evaluated using plantar test equipment that focuses a radiative source on the paw of the animal, estimating the latency time from irradiation to the withdrawal of the paw. In this model, we compared the analgesic efficacy of the peptides (1 mg/kg, i.m.) with ibuprofen (1 mg/kg, i.m.) 24 hours after the injection of CFA. We also monitored the thermal sensitivity in the contralateral paw (injected with vehicle of CFA) after 1hr, 2hrs, 4hrs and 6hrs post-CFA. Figure 1 shows that the peptides reduced the thermal hyperalgesia 2 hours after administration. Therefore, the peptides of the invention possess analgesic/anti-inflammatory activity in the model of chronic pain. Figure 1.

## Claims

1. Peptide with general formula (I)
R₁-AA-R₂ (I)
its stereoisomers, mixtures thereof, and its cosmetically and pharmaceutically acceptable salts, **wherein**
AA is a sequence of adjacent amino acids selected from the group consisting of SEQ ID No.4, SEQ ID No.8, SEQ ID No.9, SEQ ID No.11, SEQ ID No.14, SEQ ID No.15, SEQ ID No. 16, SEQ ID No.17, SEQ ID No.18, SEQ ID No.19, SEQ ID No.20, SEQ ID No.21, SEQ ID No.22, SEQ ID No.23, SEQ ID No.24, SEQ ID No.25 and SEQ ID No.26;
R₁ is selected from the group consisting of H, a polyethylene glycol polymer 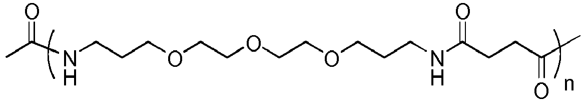 wherein n can range between 1 and 5,
and R₅-C(O)-, wherein R₅ is a substituted or non-substituted non-cyclic aliphatic group of C₁ to C₂₄, or a substituted or non-substituted alicyclyl group of C₁ to C₂₄; and
R₂ is selected from the group consisting of -NR₃R₄ and -OR₃, wherein R₃ and R₄ are selected independently from the group consisting of H, substituted or non-substituted non-cyclic aliphatic group of C₁ to C₂₄ and substituted or non-substituted alicyclyl group of C₁ to C₂₄;
for use in the treatment of pain and/or inflammation.

2. Peptide for use according to claim 1, **wherein** R₁ is selected from the group consisting of H, acetyl, *tert*-butanoyl, hexanoyl, 2-methylhexanoyl, cyclohexancarboxyl, octanoyl, decanoyl, lauroyl, miristoyl, palmitoyl, stearoyl, oleoyl and linoleoyl.

3. Peptide for use according to claim 1, **wherein** R₃ and R₄ are selected from the group consisting of H, methyl, ethyl, hexyl, dodecyl and hexadecyl.

4. Peptide for use according to any one of claims 1 to 3, **wherein** the peptide with general formula (I), its stereoisomers, mixtures thereof, or its cosmetically or pharmaceutically acceptable salts, is incorporated into a delivery or a sustained release system selected from the group consisting of liposomes, millicapsules, microcapsules, nanocapsules, sponges, vesicles, micelles, millispheres, microspheres, nanospheres, lipospheres, microemulsions, nanoemulsions, milliparticles, microparticles and nanoparticles.

5. Peptide for use according to any one of claims 1 to 4, **which** is adsorbed on an organic polymer or solid mineral carrier selected from a group consisting of talc, bentonite, silica, starch or maltodextrin.

6. Peptide for use according to any one of claims 1 to 5, **which** is contained in a formulation selected from the group consisting of creams, multiple emulsions, anhydrous compositions, aqueous dispersions, oils, milks, balms, foams, lotions, gels, hydroalcoholic solutions, liniments, sera, soaps, shampoos, unguents, mousses, ointments, powders, bars, pencils, sprays and aerosols.

7. Peptide for use according to any one of claims 1 to 6, **which** is incorporated into a fabric, a non-woven fabric or a medical device.

8. Peptide for use according to any one of claims 1 to 7, **which** is contained in a composition comprising an effective amount of at least one active ingredient selected from the group consisting of an antioxidant agent, a NO-synthase inhibitor, a skin relaxing agent, an anti-inflammatory agent, an analgesic agent, an antimicrobial agent, an antifungal agent, or mixtures thereof.

9. Peptide for use according to any one of claims 1 to 8, **which** is administered by topical, enteral or parenteral route.

10. Peptide for use according to claim 1, **wherein** the pain and/or inflammation are selected from the group consisting of neuropathic pain, inflammatory pain, visceral pain, abdominal pain, pain of the digestive system, pain of the respiratory system, pain of the urogenital system, pain of the endocrine system, heart pain, pancreatic pain, intestinal pain, stomach pain, spleen pain, blood vessel pain, irritable bowel syndrome, tension headache pain, headache associated with sinusitis, migraine, eye pain, dry eye syndrome, post-operative pain, post-operative pain due to surgical incisions, post-operative pain due to the insertion of implants in bone, post-operative pain due to bone replacement, post-operative pain due to infection, pain due to cancer, pain due to bone cancer, pain associated with benign bone tumors, pain associated with osteoid osteoma, pain associated with osteoblastomas, pain due to cancer treatment, musculoskeletal pain, fibromyalgia, nerve pain, neck pain associated with cervical dystonia, back pain, lumbago, sciatica, neurogenic inflammation, skin irritation, sensitive skin, atopic dermatitis, contact dermatitis, diaper dermatitis, eczema, arthritis, rheumatoid arthritis, osteoarthritis, post-herpetic neuralgia, peripheral neuropathies, phantom pain, allodynia, pain due to carpal tunnel syndrome, burning pain, paresthesia, facial pain, trigeminal neuralgia, neuropathic pain due to diabetes, pain associated with tattooing or tattoo removal, pain due to bunions, testicular pain, myofascial pain, urinary bladder pain, urinary tract pain, vulvar pain, vaginal pain, scrotal pain, perineal pain, pelvic pain, pain or skin irritation after surgery, after treatment with pulsed light therapy (IPL, Intense Pulse Light), after treatment with pulsed monochromatic light therapy (laser), after treatment with chemical exfoliating agents or after overexposure to aggressive external agents.

## Patentansprüche

1. Peptid der allgemeinen Formel (I)
R₁-AA-R₂ (I)
seine Stereosisomere, Mischungen davon und seine kosmetisch und pharmazeutisch verträglichen Salze, **wobei**
AA eine Sequenz von benachbarten Aminosäuren ist, die ausgewählt ist aus der Gruppe bestehend aus SEQ ID No.4, SEQ ID No.8, SEQ ID No.9, SEQ ID No.11, SEQ ID No.14, SEQ ID No.15, SEQ ID No. 16, SEQ ID No.17, SEQ ID No.18, SEQ ID No.19, SEQ ID No.20, SEQ ID No.21, SEQ ID No.22, SEQ ID No.23, SEQ ID No.24, SEQ ID No.25 und SEQ ID No.26;
R₁ ausgewählt ist aus der Gruppe bestehend aus H, einem Polyethylenglycolpolymer 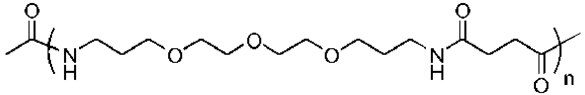 wobei n im Bereich zwischen 1 und 5 liegen kann,
und R₅-C(O)-, wobei R₅ eine substituierter oder nichtsubstituierte nichtcyclische aliphatische Gruppe von C₁ bis C₂₄, oder eine substituierte oder nichtsubstituierte alicyclische Gruppe von C₁ bis C₂₄ ist; und
R₂ ausgewählt ist aus der Gruppe bestehend aus -NR₃R₄ und -OR₃, wobei R₃ und R₄ unabhängig ausgewählt sind aus der Gruppe bestehend aus H, einer substituierten oder nichtsubstituierten nichtcyclischen aliphatischen Gruppe von C₁ bis C₂₄und einer substituierten oder nichtsubstituierten alicyclischen Gruppe von C₁ bis C₂₄;
zur Verwendung bei der Behandlung von Schmerzen und/oder Entzündungen.

2. Peptid zur Verwendung nach Anspruch 1, **wobei** R₁ ausgewählt ist aus der Gruppe bestehend aus H, Acetyl, tert-Butanoyl, Hexanoyl, 2-Methylhexanoyl, Cyclohexancarboxyl, Octanoyl, Decanoyl, Lauroyl, Miristoyl, Palmitoyl, Stearoyl, Oleoyl und Linoleoyl.

3. Peptid zur Verwendung nach Anspruch 1, **wobei** R₃ und R₄ ausgewählt sind aus der Gruppe bestehend aus H, Methyl, Ethyl, Hexyl, Dodecyl und Hexadecyl.

4. Peptid zur Verwendung nach einem der Ansprüche 1 bis 3, **wobei** das Peptid mit der allgemeinen Formel (I), seine Stereosisomere, Mischungen davon oder seine kosmetisch und pharmazeutisch verträglichen Salze in ein Abgabe- oder Retardsystem aufgenommen wird, das ausgewählt ist aus der Gruppe bestehend aus Liposomen Millikapseln, Mikrokapseln, Nanokapseln, Schwämmen, Vesikeln, Mizellen, Mikrosphären, Mikrosphären, Nanosphären, Liposphären, Mikroemulsionen, Nanoemulsionen, Millipartikeln, Mikropartikeln und Nanopartikeln.

5. Peptid zur Verwendung nach einem der Ansprüche 1 bis 4, **das** auf einem organischen Polymer- oder festen Mineralienträger adosbirert wird, der ausgewählt ist aus der Gruppe bestehend aus Talk, Bentonit, Siliciumdioxid, Stärke oder Maltodextrin.

6. Peptid zur Verwendung nach einem der Ansprüche 1 bis 5, **das** in einer Formulierung enthalten ist, die ausgewählt ist aus der Gruppe bestehend aus Cremes, Mehrfachemulsionen, wasserfreien Zusammensetzungen, wässrigen Dispersionen, Ölen, Milch, Balsamen, Schäumen, Lotionen, Gelen, hydroalkoholischen Lösungen, Linimenten, Seren, Seifen, Shampoos, Salben, Mousses, Salben, Pudern, Stifte, Kosmetikstifte, Sprays und Aerosolen.

7. Peptid zur Verwendung nach einem der Ansprüche 1 bis 6, **das**in einen Stoff, ein Vliesstoff oder eine medizinische Vorrichtung aufgenommen wird.

8. Peptid zur Verwendung nach einem der Ansprüche 1 bis 7, **das** in einer Zusammensetzung enthalten ist, die eine wirksame Menge mindestens eines Wirkstoffs umfasst, der ausgewählt ist aus der Gruppe bestehend aus einem Antioxidationsmittel, einem NO-Synthase-Inhibitor, einem Hautentspannungsmittel, einem entzündungshemmenden Mittel, einem Analgetikum, einem antimikrobiellen Mittel, ein antimykotisches Mittel oder Mischungen davon.

9. Peptid zur Verwendung nach einem der Ansprüche 1 bis 8, **das** durch topischen, enteralen oder parenteralen Weg verabreicht wird.

10. Peptid zur Verwendung nach Anspruch 1, **wobei** die Schmerzen und/oder Entzündung ausgewählt sind aus der Gruppe bestehend aus neuropathischen Schmerzen, Entzündungsschmerzen, viszeralen Schmerzen, Bauchschmerzen, Schmerzen des Verdauungssystems, Schmerzen der Atemwege, Schmerzen des Urogenitalsystems, Schmerzen des endokrinen Systems, Herzschmerzen, Bauchspeicheldrüsenschmerzen, Darmschmerzen, Magenschmerzen, Milzschmerzen, Blutgefäßschmerzen, Reizdarmsyndrom, Spannungskopfschmerzen, mit Sinusitis verbundenen Kopfschmerzen, Migräne, Augenschmerzen, trockenem Augensyndrom, postoperativen Schmerzen, postoperativen Schmerzen aufgrund chirurgischer Schnitte, postoperativen Schmerzen aufgrund des Einsetzens von Implantaten in Knochen, postoperativen Schmerzen aufgrund von Knochenersatz, postoperativen Schmerzen aufgrund von Infektionen, Schmerzen aufgrund von Krebs, Schmerzen aufgrund von Knochenkrebs, Schmerzen im Zusammenhang mit gutartigen Knochentumoren, Schmerzen im Zusammenhang mit Osteoidosteom, Schmerzen im Zusammenhang mit Osteoblastomen, Schmerzen aufgrund von Krebsbehandlung, Muskel-Skelett-Schmerzen, Fibromyalgie, Nervenschmerzen, Nackenschmerzen, die mit zervikaler Dystonie in Zusammenhang stehen, Rückenschmerzen, Hexenschuss, Ischias, neurogener Entzündung, Hautreizung, empfindlicher Haut, atopischer Dermatitis, Kontaktdermatitis, Windeldermatitis, Ekzem, Arthritis, rheumatoider Arthritis, Osteoarthritis, postherpetischer Neuralgie, peripheren Neuropathien, Phantomschmerzen, Allodynie, Schmerzen aufgrund des Karpaltunnelsyndroms, brennenden Schmerzen, Parästhesie, Gesichtsschmerzen, Trigeminusneuralgie, neuropathischen Schmerzen aufgrund von Diabetes, Schmerzen im Zusammenhang mit Tätowierung oder Entfernung von Tätowierungen, Schmerzen aufgrund von Hallux valgus, Hodenschmerzen, myofaszialen Schmerzen, Harnblasenschmerzen, Harnwegsschmerzen, Vulvaschmerzen, vaginalen Schmerzen, skrotalen Schmerzen, Dammschmerzen, Beckenschmerzen, Schmerzen oder Hautirritationen nach einer Operation, nach Behandlung mit Pulslichttherapie (IPL, Intense Pulse Light), nach Behandlung mit gepulster monochromatischer Lichttherapie (Laser), nach Behandlung mit chemischen Peelingmitteln oder nach Überexposition gegenüber aggressiven externen Agenten.

## Revendications

1. Peptide ayant la formule générale (I)
R₁-AA-R₂ (I)
ses stéréoisomères, ses mélanges, et ses sels cosmétiquement et pharmaceutiquement acceptables, **dans lequel**
AA est une séquence d'acides aminés adjacents choisie dans le groupe consistant en SEQ ID No. 4, SEQ ID No. 8, SEQ ID No. 9, SEQ ID No. 11, SEQ ID No. 14, SEQ ID No. 15, SEQ ID No. 16, SEQ ID No. 17, SEQ ID No. 18, SEQ ID No. 19, SEQ ID No. 20, SEQ ID No. 21, SEQ ID No. 22, SEQ ID No. 23, SEQ ID No. 24, SEQ ID No. 25 et SEQ ID No. 26;
R₁ est choisi dans le groupe consistant en H, un polymère polyéthylène glycol 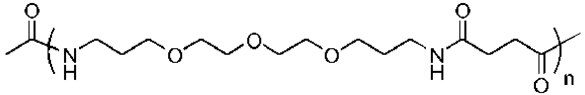 dans lequel n peut être compris entre 1 et 5,
et R₅-C(O)-, dans lequel R₅ est un groupe aliphatique non-cyclique substitué ou non-substitué de C₁ à C₂₄, ou un groupe alicyclyle substitué ou non-substitué de C₁ à C₂₄; et
R₂ est choisi dans le groupe consistant en -NR₃R₄ et -OR₃, dans lequel R₃ et R₄ sont indépendamment choisis dans le groupe consistant en H, un groupe aliphatique non-cyclique substitué ou non-substitué de C₁ à C₂₄ et un groupe alicyclyle substitué ou non-substitué de C₁ à C₂₄ ;
pour son utilisation dans le traitement de la douleur et/ou de l'inflammation.

2. Peptide pour son utilisation selon la revendication 1, **dans lequel** R₁ est choisi dans le groupe consistant en H, acétyle, *tert*-butanoyle, hexanoyle, 2-méthylhexanoyle, cyclohexancarboxyle, octanoyle, décanoyle, lauroyle, miristoyle, palmitoyle, stéaroyle, oléoyle et linoléoyle.

3. Peptide pour son utilisation selon la revendication 1, **dans lequel** R₃ et R₄ sont choisis dans le groupe qui consiste en H, méthyle, éthyle, hexyle, dodécyle et hexadécyle.

4. Peptide pour son utilisation selon l'une quelconque des revendications 1 à 3, **dans lequel** le peptide ayant la formule générale (I), ses stéréoisomères, ses mélanges, ou ses sels cosmétiquement ou pharmaceutiquement acceptables, est incorporé dans un système de délivrance ou de libération prolongée choisi dans le groupe consistant en liposomes, millicapsules, microcapsules, nanocapsules, éponges, vésicules, micelles, millisphères, microsphères, nanosphères, liposphères, microémulsions, nanoémulsions, milliparticules, microparticules et nanoparticules.

5. Peptide pour son utilisation selon l'une quelconque des revendications 1 à 4, **qui** est absorbé sur un polymère organique ou véhicule minéral solide choisi dans un groupe consistant en talc, bentonite, silice, amidon ou maltodextrine.

6. Peptide pour son utilisation selon l'une quelconque des revendications 1 à 5, **qui** est contenu dans une formulation choisie dans le groupe consistant en crèmes, émulsions multiples, compositions anhydres, dispersions aqueuses, huiles, laits, baumes, substances mousseuses, lotions, gels, solutions hydroalcooliques, liniments, sérums, savons, shampooings, onguents, mousses, pommades, poudres, bâtons, crayons, sprays et aérosols.

7. Peptide pour son utilisation selon l'une quelconque des revendications 1 à 6, **qui** est incorporé dans un tissu, un tissu non tissé ou un dispositif médical.

8. Peptide pour son utilisation selon l'une quelconque des revendications 1 à 7, **qui** est contenu dans une composition comprenant une quantité efficace d'au moins un ingrédient actif choisi dans le groupe consistant en un agent antioxydant, un inhibiteur de l'oxyde nitrique synthase, un agent relaxant pour la peau, un agent anti-inflammatoire, un agent analgésique, un agent anti-microbien, un agent antifongique, ou des mélanges de ces derniers.

9. Peptide pour son utilisation selon l'une quelconque des revendications 1 à 8, **qui** est administré par voie topique, entérale ou parentérale.

10. Peptide pour son utilisation selon la revendication 1, **dans lequel** la douleur et/ou l'inflammation sont choisies dans le groupe consistant en douleur neuropathique, douleur inflammatoire, douleur viscérale, douleur abdominale, douleur du système digestif, douleur du système respiratoire, douleur du système urogénital, douleur du système endocrinien, douleur cardiaque, douleur pancréatique, douleur intestinale, douleur à l'estomac, douleur de la rate, douleur des vaisseaux sanguins, syndrome du côlon irritable, douleurs de céphalées de tension, céphalées associées à la sinusite, migraine, douleur oculaire, syndrome de l'oeil sec, douleur post-opératoire, douleur post-opératoire due aux incisions chirurgicales, douleur post-opératoire due à l'insertion d'implants osseux, douleur post-opératoire due au remplacement osseux, douleur post-opératoire due à l'infection, douleur due au cancer, douleur due au cancer des os, douleur associée aux tumeurs bénignes des os, douleur associée à l'ostéome ostéoïde, douleur associée aux ostéoblastomes, douleur due au traitement du cancer, douleur musculosquelettique, fibromyalgie, douleur des nerfs, douleur à la nuque associée à la dystonie cervicale, douleur dorsale, lumbago, sciatique, inflammation neurogène, irritation de la peau, peau sensible, dermatite atopique, dermatite de contact, dermite du siège, eczéma, arthrite, arthrite rhumatoïde, ostéoarthrite, névralgie post-herpétique, neuropathies périphériques, douleur fantôme, allodynie, douleur due au syndrome du canal carpien, douleur cuisante, paresthésie, douleur faciale, névralgie trigéminale, douleur neuropathique due aux diabètes, douleur associée au tatouage ou à l'élimination du tatouage, douleur due aux oignons, douleur testiculaire, douleur myofasciale, douleur de la vessie urinaire, douleur de la tract urinaire, douleur vulvaire, douleur vaginale, douleur scrotale, douleur périnéale, douleur pelvienne, douleur ou irritation de la peau après la chirurgie, après le traitement avec la thérapie à la lumière pulsée (IPL, Intense Pulse Light), après le traitement avec la thérapie à la lumière monochromatique pulsée (laser), après le traitement avec des agents exfoliants chimiques ou après la surexposition aux agents externes agressifs.
